# EUROPEAN PATENT SPECIFICATION

(11) **EP 0 828 750 B1**
(45) Date of publication and mention of the grant of the patent: **17.07.2013**
(21) Application number: 96919015.6
(22) Date of filing: 30.05.1996
(51) Int. Cl.: C07H 19/06

(54) **PALLADIUM CATALYZED NUCLEOSIDE MODIFICATION METHODS USING NUCLEOPHILES AND CARBON MONOXIDE**
PALLADIUMKATALYSIERTE VERFAHREN ZUR MODIFIKATION VON NUKLEOSIDEN UNTER VERWENDUNG VON NUKLEOPHILEN UND KOHLENMONOXID
PROCEDES DE MODIFICATION DE NUCLEOSIDES PAR CATALYSE AU PALLADIUM UTILISANT DES NUCLEOPHILES ET DU MONOXYDE DE CARBONE

(30) Priority: 02.06.1995 US 459073; 02.06.1995 US 458421
(43) Date of publication of application: 18.03.1998
(73) Proprietor: EC Technology LLC, Denver, Colorado 80206 (US)
(72) Inventor: TU, Chi, Louisville, CO 80027 (US); DEWEY, Torin, Boulder, CO 80303 (US); EATON, Bruce, Boulder, CO 80302 (US)
(74) Representative: Clegg, Richard Ian
(86) International application number: PCT/US1996/008026
(87) International publication number: WO 1996/038460

(56) References cited:
- EP-A2- 0 208 550
- US-A- 5 420 276
- US-A- 5 428 149
- H. HAYAKAWA ET AL.: "Lithiation of 5,6-dihydrouridine: a new route to 5-substituted uridines" TETRAHEDRON, vol. 41, 1985, pages 1675-1683, XP002452346
- G.J. GROUCH, B.E. EATON: "Synthesis of 2'-deoxyuridine nucleosides with appended 5-position carbonyl cross-linking groups" NUCLESIDES AND NUCLEOTIDES, vol. 13, 1994, pages 939-944, XP008084125
- MATSUDA ET AL: "Nucleosides and nucleotides. XXVII. Synthesis of 2- and 8-cyanoadenosines and their derivatives" CHEMICAL AND PHARMACEUTICAL BULLETIN, PHARMACEUTICAL SOCIETY OF JAPAN, TOKYO, JP, vol. 27, no. 1, 1979, pages 183-192, XP002127436 ISSN: 0009-2363
- H. HAYAKAWA ET AL.: "Direct C-8 lithiation of naturally-occurring purine nucleosides. A simple method for the synthesis of 8-carbon-substituted purine nucleosides" CHEM. PHARM. BULL., vol. 35, 1987, pages 72-79, XP002452347
- N. CONG-DANH ET AL.: "Modifications de la position C-8 de purines nucleosides par action de reactifs electrophiles sur leurs derives silyles et lithes" TETRAHEDRON LETT., vol. 26, 1979, pages 2385-2388, XP002452348
- M. MAEDA ET AL.: "Studies on chemical alterations of nucleic acid and their components - VII." TETRAHEDRON, vol. 30, 1974, pages 2677-1682, XP002452349
- A. HOLY: "Nucleic acid components and their analogues. CXLVII. Preparation of 5-ethoxycarbonyluridine, 5-carboxyuridine and their nucleotide derivatives" COLLECTION CZECHOSLOV. CHEM. COMMUN., vol. 37, 1972, pages 1555-1576, XP002452350
- D.C. AGATHOCLEOUS, G. SHAW: "Purines, pyrimidines and imidazoles. Part 66. New synthesis of some uridine and N-alkoxycarbonyl 5-carboxamides, N-carbamoyl 5-carboxamides and 5-carboxamides" J. CHEM. SOC. PERKIN TRANS. 1, 1991, pages 2317-2321, XP002452351
- T.M. DEWEY ET AL.: "New uridine derivatives for systematic evolution of RNA ligands by exponential enrichment" J. AM. CHEM. SOC., vol. 117, 1995, pages 8474-8475, XP002452352

## Description

### FIELD OF THE INVENTION

This invention relates to the field of nucleic acid chemistry, specifically to a process for preparing modified nucleosides. The nucleosides can be pyrimidines or purines. The pyrimidine compounds of the invention can be modified at the 5-, or 6-position of the pyrimidine ring. The purine compounds of the invention can be modified at the 2-, 6- or 8-position of the purine ring. Most preferably, the invention includes a process for preparing 8-position modified purine compounds and 5-position modified pyrimidine compounds. The present invention also includes modified nucleosides produced by the method. The modified nucleosides produced according to methods of the present invention find use as anti-viral, anti-bacterial, anti-fungal or anti-neoplastic agents or as part of an oligonucleotide.

### BACKGROUND OF THE INVENTION

Until quite recently, the consideration of oligonucleotides in any function other than strictly informational was not known. Despite the fact that certain oligonucleotides were known to have interesting structural possibilities (e.g., t-RNAs) and other oligonucleotides were bound specifically by polypeptides in nature, very little attention had been focused on the non-informational capacities of oligonucleotides. For this reason, among others, little consideration had been given to using oligonucleotides as pharmaceutical compounds.

There are currently at least three areas of exploration that have led to serious studies regarding the use of oligonucleotides as pharmaceuticals. In the most advanced of the fields, antisense oligonucleotides are utilized to bind to certain regions in an organism to prevent the expression of proteins or to block various cell functions. The discovery of RNA species with catalytic functions -- ribozymes -- has led to the consideration of RNA species that serve to perform intracellular reactions that will achieve desired effects. And lastly, the discovery of the SELEX process (Systematic Evolution of Ligands by EXponential Enrichment) has shown the research community that oligonucleotides can be identified that will bind to almost any biologically interesting target.

The use of antisense oligonucleotides as a method for controlling gene expression and the potential for using oligonucleotides as pharmaceutical materials has prompted investigations into the introduction of a number of chemical modifications into oligonucleotides to increase their therapeutic activity. Such modifications are designed to increase cell penetration of the oligonucleotides, to stabilize them from nucleases and other enzymes that degrade or interfere with the structure or activity of the oligonucleotide analogs in the body, to enhance their binding to targeted nucleic acids, to provide a mode of disruption (terminating event) once sequence-specifically bound to targeted nucleic acids, and to improve their pharmacokinetic properties. For example, PCT Patent Application Publication WO 91/14696, entitled: Oligonucleotide-Transport Agent Disulfide Conjugates, describes a method for chemically modifying antisense oligonucleotides to enhance entry into a cell.

A variety of methods have been used to render oligonucleotides resistant to degradation by exonucleases. PCT Patent Application Publication WO 90/15065, entitled: Exonuclease-Resistant Oligonucleotides and Methods for Preparing the Same, describes a method for making exonuclease-resistant oligonucleotides by incorporating two or more phosphoramidite and phosphoromonothionate and/or phosphorodithionate linkages at the 5' and/or 3' ends of the oligonucleotide. PCT Patent Application Publication WO 91/06629, entitled: Oligonucleotide Analogs with Novel Linkages, describes oligonucleotide compounds with one or more phosphodiester linkages between adjacent nucleotides replaced by a formacetal/ketal type linkage which are capable of binding RNA or DNA.

A common strategy for stabilization of RNA against endonucleolytic cleavage is to modify the 2'-position of ribonucleotides. Interference with base recognition by enzymes can be used to approach stabilization against base-specific endonucleolytic cleavage. Several strategies for this modification are known, including modification with 2'-amino and 2'-fluoro (Hobbs et al. (1973) Biochemistry 12:5138; Guschlbauer et al. (1977) Nucleic Acids Res. 4:1933), and 2'-OCH₃ (Shibahara et al. (1987) 15:4403; Sproat et al. (1989) Nucleic Acids Res. 17:3373). PCT Patent Application Publication WO 91/06556, entitled: 2' Modified Oligonucleotides, describes nuclease-resistant oligomers with substituents at the 2' position. PCT Patent Application Publication WO 91/10671, entitled: Compositions and Methods for Detecting and Modulating RNA Activity and Gene Expression, describes antisense oligonucleotides chemically modified at the 2' position and containing a reactive portion capable of catalyzing, alkylating, or otherwise effecting the cleavage of RNA, a targeting portion, and a tether portion for connecting the targeting and reactive portions.

The 5-position of pyrimidines may also be chemically modified. The introduction of modifications at the C-5 position of pyrimidines may be envisioned to interfere with the recognition by pyrimidine specific endonucleases. However, this concept is not as clear cut as the modification of the 2'-position of ribonucleotides.

The use of palladium to catalyze carbon-carbon bond formation at the 5 position of pyrimidine nucleosides is known. A superior method for 5-position modification of pyrimidines is described in United States Patent Number 5,428,149, entitled "Method for Palladium Catalyzed Carbon-Carbon Coupling and Product," which is herein incorporated by reference in its entirety. The first examples of 5-position pyrimidine modifications were demonstrated by Bergstrom (Bergstrom et al. (1976) J. Am. Chem. Soc. 98:1587, (1978) J. Org. Chem. 43:2870, (1981) J. Org. Chem. 46:1432 and 2870, (1982) J. Org. Chem. 47:2174) and Daves (Arai and Daves (1978) J. Am. Chem. Soc., 100:287; Lee and Daves (1983) J. Org. Chem. 48:2870). Bergstrom and Daves used 5-mercurial-deoxyuridine compounds, the same as those used by Dreyer and Dervan ((1985) Proc. Natl. Acad. Sci. USA 82:968), to tether functional groups to oligonucleotides.

One method for simple carbon-carbon coupling reactions to the 5-position of uridines is described in the work of Crisp (1989) Syn. Commun. 19:2117. Crisp forms deoxyuridines functionalized at the 5-position by reacting protected 5-iodo-2'-deoxyuridine with alkenylstannanes in acetonitrile in the presence of a Pd (II) catalyst.

To date, very little work has been done to modify purine nucleosides using palladium catalysis. Aeroschot et al., ((1993) J. Med. Chem 36:2938-2942) report that 2-, 6-, and 8-halogenated adenosines can be modified with symmetric organotin reagents. However, symmetric organotin compounds are not widely available. Sessler et al., ((1993) J. Am. Chem. 115:10418-10419) describe the arylation of protected 8-bromoguanosine with 4-tributyltinbenzaldehyde. However, using this procedure, a significant amount of starting material (28%) was unreacted. A superior method for modifying purine nucleosides using palladium catalysts is described in United States Patent Application Serial Number 08/347,600, filed December 1, 1994, entitled "Purine Nucleoside Modification by Palladium Catalyzed Methods", which is herein incorporated by reference in its entirety.

A simple modification of Stille type carbonylative coupling conditions has been reported to provide high yield reactions giving new carbonyl appended 2'-deoxyuridine derivatives useful for chemical crosslinking; in particular the combination of Pd (II) acetate with three equivalents of triphenylphosphine and copper (I) iodide resulted in improved catalytic performance (Nucleosides and Nucleotides (1994) 13(4): 939-944).

Additionally, very little work has been done in the area of palladium catalyzed amidations. Schoenberg, et al. (J. Org. Chem. (1974) 39:3327) describe amidation of aryl and alkenyl halides, however, this work does not include nucleoside substrates or the use of a PdL₄ catalyst.

SELEX (Systematic Evolution of Ligands for EXponential Enrichment) is a method for identifying and producing nucleic acid ligands, termed "nucleic acid antibodies", e.g., nucleic acids that selectively bind to target molecules (Tuerk and Gold (1990) Science 249:505). The method involves selection from a mixture of candidates and step-wise iterations of structural improvement, using the same general selection theme, to achieve virtually any desired criterion of affinity and selectivity. Starting from a mixture of nucleic acids, the method includes steps of contacting the mixture with the target under conditions favorable for interaction, partitioning non-interacting nucleic acids from those nucleic acids which have interacted with the target molecules, dissociating the nucleic acid-target pairs, amplifying the nucleic acids dissociated from the nucleic acid-target pairs to yield a mixture of nucleic acids enriched for those which interact with the target, then reiterating the steps of interacting, partitioning, dissociating and amplifying through as many cycles as desired.

The methods of the present invention may be combined with SELEX to produce nucleic acids containing modified nucleotides. The presence of modified nucleotides may result in nucleic acids with an altered structure exhibiting an increased capacity to interact with target molecules. The steric and electronic influence of modified nucleosides may also act to prevent nuclease degradation. Incorporation of modified nucleotides into oligonucleotides is well known to those skilled in the art (Dewey, T. et al., J. Amer. Chem. Soc. (1995) 117:8474-8475; Walker G. C. et al., Biochemistry (1975) 14:817-823; Connolly, B. A., pp 155-183 In Oligonucleotides and Analogues: A Practical Approach (1991)(editor F. Eckstein) IRL Press, New York).

### BRIEF SUMMARY OF THE INVENTION

The present invention provides a method for the preparation of a modified nucleoside according to claim 1. The present invention also provides a compound according to claim 16, as well as such compounds for use in therapy and in the preparation of a medicament for use in inhibiting or treating cytomegalovirus activity according to claim 24 or claim 25. The present invention also provides an oligoribonucleotide or oligodeoxyribonucleotide according to claim 22.

The present invention therefore includes a novel method for introducing chemical moieties at various positions of nucleoside rings utilizing a palladium catalyst and a nucleophile and carbon monoxide. Preferably, the modifications are at the 5- or 6-position of a pyrimidine ring or at the 2-, 6-, or 8-positions of the purine ring. Most preferably the modifications are at the 5-position of the pyrimidine ring and at the 8-position of the purine ring. Particularly referred modifications of the nucleoside ring include the introduction of an amide or ester moiety. For the preferred modifications, the nucleophile is a primary or secondary amine.

This invention includes a reaction scheme for producing a wide variety of modified nucleoside molecules. A key element in the production of the modified nucleosides is the use of a palladium catalyst in conjunction with a nucleophile and carbon monoxide.

More specifically, the invention provides a method for the preparation of a modified nucleoside comprising the steps of reacting a nucleoside starting material containing a leaving group attached to a carbon atom of the nucleoside starting material with a nucleophile and carbon monoxide in the presence of a palladium catalyst; and isolating the modified nucleoside. The modified nucleosides produced by this method are also included in the invention.

This invention further includes a method of preparing stabilized nucleic acids wherein the modified nucleoside is coupled to a sugar modified at the 2'-position or the 3'-position.

The modified nucleosides of the invention have many uses including, but not limited to, use as anti-viral, anti-bacterial, anti-fungal, or anti-neoplastic agents and use as part of an oligonucleotide.

### DETAILED DESCRIPTION OF THE INVENTION

The present invention includes a method for modifying a nucleoside ring by reacting a nucleoside starting material containing a leaving group attached to a carbon atom of said nucleoside starting material with a nucleophile and carbon monoxide in the presence of a palladium catalyst; and isolating said modified nucleoside, wherein said nucleophile is an amine, alcohol or thiol. The invention includes the modifications of both pyrimidines and purines. The pyrimidines have the following structures and conventional numbering:

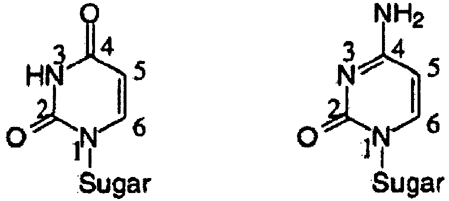

The pyrimidine ring can be modified at the 5- or 6-position; most preferably the 5-position is modified. The purines have the following structures and conventional numbering:

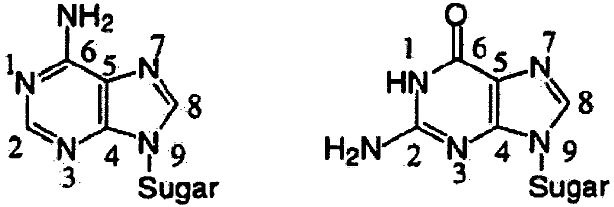

The purine can be modified at positions 2-, 6- and 8- of the purine ring; most preferably the 8-position is modified. Introduction of a variety of modifications to the nucleoside ring are contemplated by this invention. However, particularly preferred modifications to the nucleoside ring include the introduction of an amide or ester moiety. In the preferred modifications, the nucleophile for the carboxyamidation reaction is a primary or secondary amine.

The present invention extends to novel compounds that can be prepared according to the methods of the present invention selected from
(A) 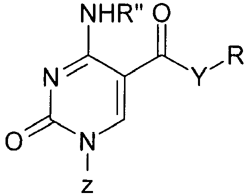 wherein
   Y is selected from the group consisting of S and NH; and
   R is selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R can optionally be part of a cyclic structure which can be aromatic, aliphatic, or heterocyclic;
   R" is selected from the group consisting of H and acyl; and
   Z is selected from the group consisting of a ribose, deoxyribose, dideoxyribose, and any combination of 2', 3', and 5' modifications thereof;
(B) 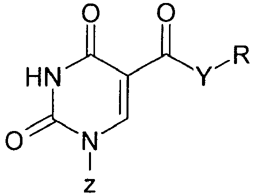 wherein
   Y is selected from the group consisting of S, NH and NR'; and
   R and R' are independently selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R and R' can optionally be part of a cyclic structure which can be aromatic, aliphatic, or heterocyclic;
   R" is selected from the group consisting of H and acyl; and
   Z is selected from the group consisting of a ribose, deoxyribose, dideoxyribose, and any combination of 2', 3', and 5' modifications thereof; and
(C) the group consisting of: 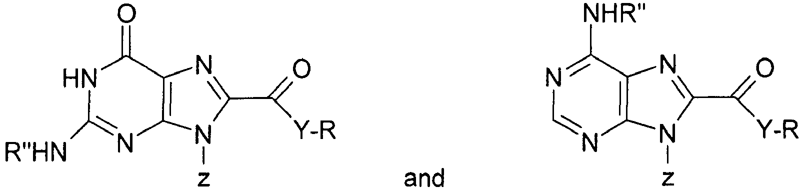 wherein
   Y is selected from the group consisting of S and NH; and
   R is independently selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R can optionally be part of a cyclic structure which can be aromatic, aliphatic, or heterocyclic;
   R" is selected from the group consisting of H and acyl; and
   Z is selected from the group consisting of a ribose, deoxyribose, dideoxyribose, and any combination of 2', 3', and 5' modifications thereof.

The present invention also includes oligonucleotides that contain one or more of the novel substituted nucleosides of this invention. The modified nucleosides produced according to methods of the present invention find use in various pharmaceutical areas, particularly as anti-virals, anti-bacterials, anti-fungals and anti-neoplastics. The present invention therefore also includes the novel compounds for use in therapy, and for inhibiting or treating cytomegalovirus activity, as well as use of the novel compounds in the preparation of a medicament for use in inhibiting or treating cytomegalovirus activity.

For example, EP 0208550 discloses the use of 5-ethynyluridine derivatives in the treatment of Cytomegalovirus (CMV).

The general reactions of the present invention can be characterized as follows:

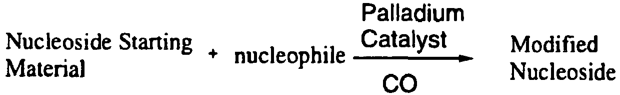

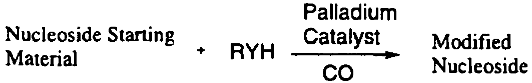

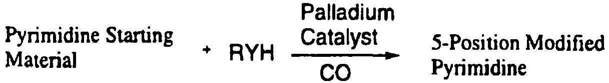

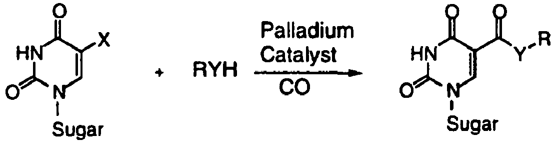

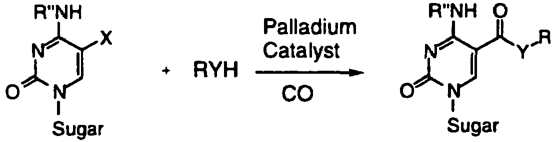

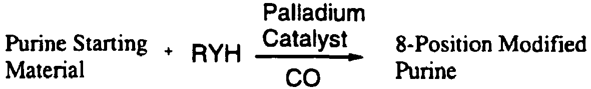

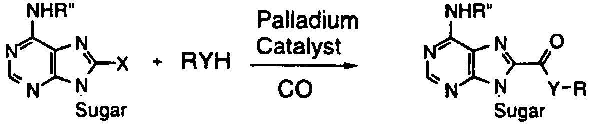

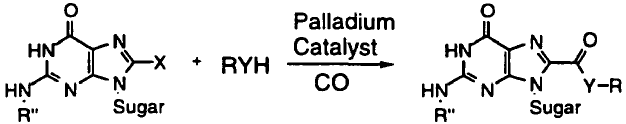

As used herein the term "modified nucleoside" is intended to encompass any nucleoside base, nucleoside, or nucleotide that may be prepared by the method of the present invention. The terms nucleoside base, nucleoside and nucleotide can be used interchangeably herein. The modified nucleosides of the invention can contain various other modifications on the base and sugar.

"Nucleoside starting material" is defined herein as any nucleoside base, nucleoside or nucleotide which has an attached acceptable leaving group (X). Nucleoside starting materials include all nucleosides, both naturally occurring and non-naturally occurring. Preferably, nucleoside starting materials include purines and pyrimidines, which include uracil, thymine, cytosine, adenine and guanine starting materials, or protected derivatives thereof. In certain embodiments, the protected derivatives include those wherein R" is dimethyl formamidine or an acyl group, preferably selected from the group consisting of isobutyryl, acetyl, phenoxyacetyl, and benzoyl. R" is H in unprotected nucleosides. The leaving group can be attached to any free carbon on the nucleoside starting material. The acceptable leaving group is displaced during the catalysis reaction and replaced by C(O)YR chemical moieties to yield the modified base, nucleoside or nucleotide of the invention. The nucleoside starting material can have a sugar moiety attached in the form of a ribose, deoxyribose, dideoxyribose and any combination of 2', 3' or 5' modifications thereof. The invention contemplates the above sugar moieties and any suitable derivatives thereof, such as a ribose or 2'-deoxyribose wherein the hydroxyl groups have been partially or fully protected. For example, the 5'-hydroxyl can be present as the mono-, di-, or tri-phosphate.

"Pyrimidine starting material" is defined herein as a pyrimidine base, pyrimidine nucleoside or pyrimidine nucleotide which has an attached acceptable leaving group (X). Pyrimidine starting materials include all pyrimidines, both naturally occurring and non-naturally occurring. Preferably, pyrimidine starting materials include uracil, thymine, and cytosine, or protected derivatives thereof. The leaving group can be attached to any free carbon on the base of the nucleoside, preferably at the 5- or 6-position. The most preferred attachment is at the 5-position of the pyrimidine ring. The acceptable leaving group is displaced during the catalysis reaction and replaced by C(O)YR chemical moieties to yield the modified pyrimidine. The pyrimidine starting material can have a sugar moiety attached in the form of a ribose, deoxyribose, dideoxyribose and any combination of 2', 3' or 5' modifications thereof. The invention contemplates the above sugar moieties and any suitable derivatives thereof, such as a ribose or 2'-deoxyribose wherein the hydroxyl groups have been partially or fully protected. For example, the 5'-hydroxyl can be present as the mono-, di-, or tri-phosphate.

"Purine starting material" is defined herein as a purine base, purine nucleoside or purine nucleotide which has an attached acceptable leaving group (X). Purine starting materials include adenine and guanine starting materials, or protected derivatives thereof. The leaving group can be attached to any carbon atom of the base of the purine, preferably at the 2-, 6-, or 8-position of the purine ring. The most preferred attachment is at the 8-position. The acceptable leaving group is displaced during the catalysis reaction and replaced by C(O)YR chemical moieties to yield the modified purine. The purine starting material can have a sugar moiety attached in the form of a ribose, deoxyribose, dideoxyribose and any combination of 2', 3' or 5' modifications thereof.

The invention contemplates the above sugar moieties and any suitable derivatives thereof, such as a ribose or 2'-deoxyribose wherein the hydroxyl groups have been partially or fully protected. For example, the 5'-hydroxyl can be present as the mono-, di-, or tri-phosphate.

"Acceptable leaving group" is defined herein as a group which is a suitable counterion for palladium(II), and is designated herein as X. In the most general embodiments of this invention, X is any of a number of acceptable leaving groups well known to those skilled in the art. Acceptable leaving groups include, but are not limited to, acetate, trifluoroacetate, trifluoromethyl sulfonate, tosylate, methane sulfonate and boronic esters and acids. In the preferred embodiment, X is a halogen, and in the most preferred embodiment X is bromine or iodine. The leaving group is attached to the carbon atom of the purine starting material by methods known to one of ordinary skill in the art.

"Nucleophile" is defined herein as would be understood by one of ordinary skill in the art. Specifically, a nucleophile is an electron rich chemical moiety capable of displacing a leaving group. Due to the nature of the catalytic reaction, the CO is inserted between said nucleoside starting material and said nucleophile. Anyone skilled in the art would recognize a useful nucleophile which could be used in a nucleophilic substitution reaction. In methods of the present invention nucleophiles consist of amines, alcohols, and thiols.

In a preferred embodiment, the general structure of the nucleophiles used in the present invention is RYH, where Y=O, S, NH, or NR'. R and R' can optionally be part of a ring-structure, which can be aromatic, aliphatic or heterocyclic. In the preferred embodiments of the invention the nucleophile (RYH) is selected from the group consisting of aliphatic or aromatic, primary or secondary amines (including cyclic amines), alcohols and thiols; wherein R and R' are selected from the group consisting of substituted or unsubstituted C1-C20 alkyl (straight-chain or branched), C2-C20 alkenyl (straight-chain or branched), aryl, heterocyclic, and natural and unnatural amino acids.

In a preferred embodiment, the nucleophile has the strucure RYH, wherein,

Y is selected from the group consisting of O, S, and NH;

R is (CH₁)ₘ(CH₃)ₙ, wherein 1 is 0, 1, or 2; m is 0-19; n is 0, 1, 2, or 3; and wherein one or more of the H are optionally substituted with =O, -OH, =NH, NH₂, +NMe₃Cl, 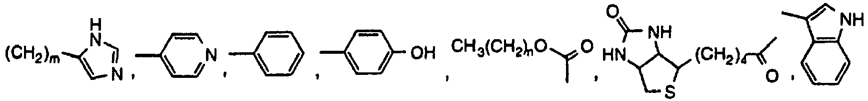 or an amino acid.

In the most preferred embodiments of the invention, the nucleophiles are selected from the following group:

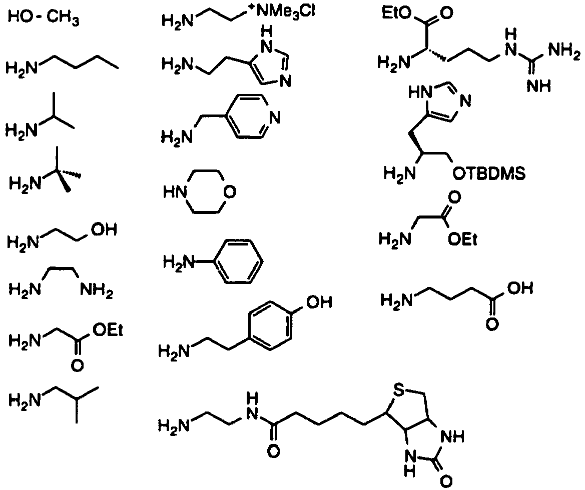

The R and R' groups of the nucleophile can include various functional groups which can be used to introduce a broad array of functional capabilities to the nucleosides prepared by this method. The nucleophile functional groups can include, among others: amides, esters, nitriles, nitros, ureas, halides, cyanates, alcohols, amines, ethers, thiols, aryl substituents, etc. as recognized by those of ordinary skill in the art. Any replacement of a hydrogen or functional group on the nucleophile is referred to as a "substitution" for the purposes of definition.

The palladium catalyst of the present invention may be characterized most generally as PdL₄ or PdL₃, where L is one of any number of commonly employed ligands of palladium. The palladium catalyst can be pre-made (e.g., PdL₄, wherein L is triphenyl phosphine, etc.) or made *in situ* from Pd(0) or Pd(II) and phosphine ligands as is known to one of ordinary skill in the art (e.g., [bis(benzylideneacetone)Pd(0)], Pd(OAc)₂, etc.). PdL₄ is the preferred palladium catalyst of the invention. It is within the skill and knowledge of those skilled in the art to recognize the various ligands that may be employed. Examples of common ligands (L) include, but are not limited to, PPh₃, (o-tol)₃P, P(*p*-C₆H₄SO₃Na)₃, CH₃CN, DMSO, N,N-dimethylformamide (DMF), 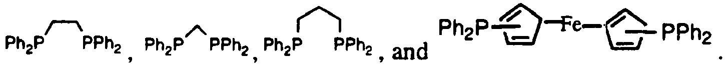 In the preferred embodiments of the catalytic species of this invention L is PPh₃ (triphenyl phosphine, or P(C₆H₅)₃) or P(*p*-C₆H₄SO₃Na)₃. The preparation of certain catalysts of the present invention is described in United States Patent Number 5,428,149, filed June 14, 1993, entitled "Method for Palladium Catalyzed Carbon-Carbon Coupling and Products" which is incorporated by reference herein.

In certain embodiments, it may be advantageous to include additional basic, non-nucleophilic components in the reaction. Examples of desirable bases include, but are not limited to, Et₃N, EtN(iPr)₂, 1,8-diazabicyclo[5.4.0]undec-7-ene (DBU), and 1,4-diazabicyclo[2.2.2]octane (DABCO). Acceptable solvents for the reaction include acetonitrile, dioxane, acetone, ethyl acetate, benzene, dimethyl formamide, dimethyl acetamide, dimethyl sulfoxide, water, THF, hexamethylphosphoramide (HMPA), and hexamethylphosphoroustriamide (HMPT). The temperature ranges for the reaction typically are between 60 and 100 degrees centrigrade, however, other suitable temperature ranges are also contemplated.

The following reactant concentrations and reaction conditions are useful in the preferred embodiments of the present invention. The nucleophile is used preferably in the range from 0.0 to 2.0 M. The concentration of the palladium catalyst can range from 0.0005 to 0.2 M. The CO pressure can range from 10 to 1000 psi. The concentration of the nucleoside starting material can range from 0.010 to 1.0 M.

The modified nucleosides and nucleotides of the present invention are contemplated for use in oligonucleotides. Standard techniques for incorporation of nucleosides into oligonucleotides can be used with the modified nucleosides of the invention. Typically, the oligonucleotides of the invention are less than 500 bases, usually less than 100 bases, and most preferably less than 50 bases. The modified nucleosides are suitable for *in vitro* transcription procedures. The oligonucleotides containing the modifed nucleosides have a number of various utilities. Specifically, the oligonucleotides interact with biological targets or have facilitating properties. The oligonucleotides can be useful in various diagnostic applications as well.

The nucleosides or nucleotides may also show antineoplastic, antibacterial, antifungal or antiviral activity. The nucleosides and nucleotides may also demonstrate other therapeutic properties. Standard assays are known to one of ordinary skill for determination of such activities. Formulation and administration routes are well known to those of ordinary skill in the art. Additionally, prodrug technology can be used as a delivery system for the nucleosides and nucleotides of the invention. Particularly, the nucleosides or nucleotides can be attached to lipids to improve pharmacology and oral availability, among other characteristics. Specifically, 5'-diacylglycero- or dialkylglycerophosphate-derivatives of the nucleosides and nucleotides of the invention are useful. These modified nucleosides and nucleotides are particularly interesting for antiviral applications. The diacylglycerophosphates of nucleosides and non-nucleosides have been used for modulation of pharmacokinetic behavior, modulation of bioavailability, and modulation of toxicity as described in United States Patent 5,223,263 which is herein incorporated by reference.

Stability towards endo-nucleolytic degradation in serum can be achieved by introducing 2'-deoxy-2'-fluoro- or 2'-deoxy-2'-aminonucleosides to the pyrimidine positions of the ligand (Pieken et al. (1991) Science 253:314). The modified nucleosides of the present invention may also be coupled with 2' substituted species that would also be useful in a variety of situations. The incorporation of halogenated nucleosides may also prove valuable for enhanced ligand-target interaction.

### EXAMPLES

The following examples are illustrative of preferred embodiments of methods of preparation and products of the invention and are not to be construed as limiting the invention thereto.

### Example 1

### Purine Modifications with Amines

The following general procedures were employed to produce the modified purines of Table I.

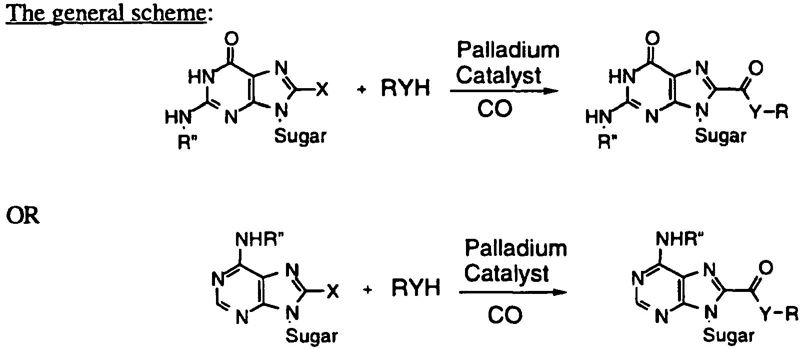

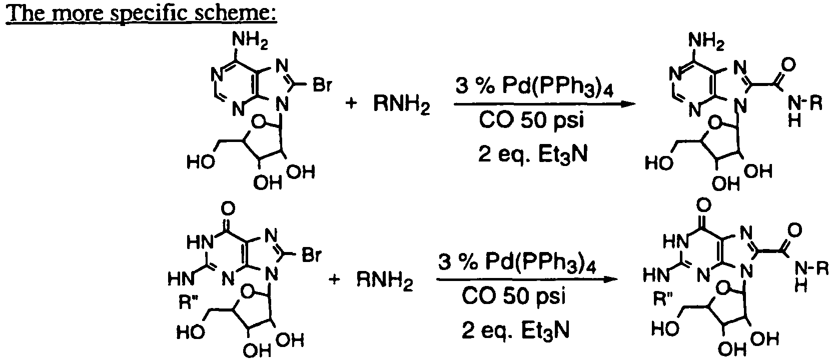

The various nucleoside base starting materials that can be used in the more specific scheme:

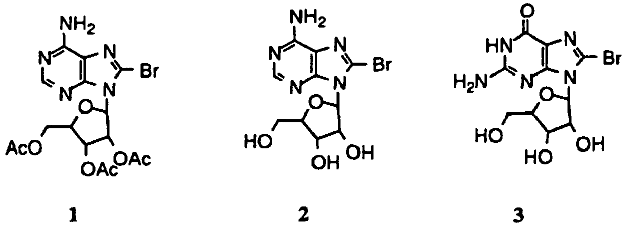

### Table I. Examples of modified purine nucleosides.

| Entry | Nucleoside | Amine Nucleophile H₂NR | Product ID | Isolated Yield (%) |
|---|---|---|---|---|
| 1 | **1** | 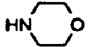 | **4** | 87^{b} |
| 2 | **1** | 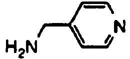 | **5** | 95 |
| 3 | **2** | 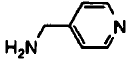 | **6** | 93 |
| 4 | **2** | 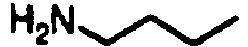 | **7** | 84^{a} |
| 5 | **2** | 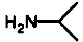 | **8** | 95 |
| 6 | **2** | 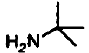 | **9** | 98 |
| 7 | **2** | 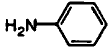 | **10** | 41 |
| 8 | **2** | 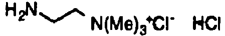 | **11** | 91 |
| 9 | **2** | 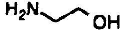 | **12** | 62^{c} |
| 10 | **2** | 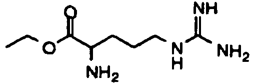 | **13** | 71 |
| 11 | **3** | 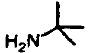 | **14** | 85 |
| 12 | **3** | 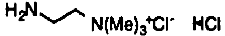 | **15** | 98 |
| 13 | **3** | 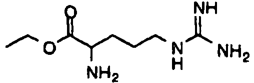 | **16** | 88 |
| 14 | **3** | 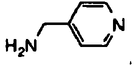 | **17** | 81 |
| 15 | 3 | 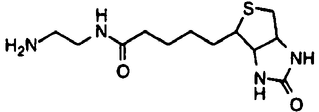 | 18 | 56 |

| | | | | |
|---|---|---|---|---|
| ^{a} The reaction produced 16 % of a side product with no CO insertion and direct coupling of the nucleophile to the nucleoside starting material. ^{b} The reaction produced 15 % of a side product with no CO insertion and direct coupling of the nucleophile to the nucleoside starting material. ^{c} The reaction produced 14 % of a side product with no CO insertion and direct coupling of the nucleophile to the nucleoside starting material and 14 % of a product resulting from CO insertion and the hydroxide serving as nucleophile rather than the amine. | | | | |

The following general procedures were followed to produce the modified purine nucleosides of Table I.
**General.** The ¹H and ¹³C NMR spectra were obtained in CD₃OD, D₂O, CDCl₃, or DMSO-d⁶ on a Bruker ARX-300 spectrometer using the deuterated solvent as an internal standard. Positive ion fast atom bomdardment mass spectra (FAB⁺) were performed at the Univ. of California at Berkeley Mass Spec. facility.
**Materials.** 8-Bromoadenosine, 8-bromoguanosine dihydrate, morpholine, n-butylamine, isopropylamine, *tert*-butylamine, aniline, 4-aminomethylpyridine, (2-aminoethyl)trimethyl-ammonium chloride, arginine ethyl ester, ethanolamine, triethylamine, DMF and N,N-dimethylacetamide (DMA) were purchased from Aldrich Chemical Company and were used as received unless otherwise noted. N-(2-Aminoethyl)biotinamide hydrobromide was purchased from Molecular Probes, Inc.
**General procedure for palladium catalyzed coupling reaction.** To a glass bomb with a Teflon valve was added the nucleoside specified in Table 1 (0.5 mmol), the amine nucleophile specified in Table 1 (1.0 mmol), Pd(PPh₃)₄ (0.0015 mmol), triethylamine (1.0 mmol) and DMF (or DMA). The glass bomb was evacuated and charged with CO (50 psi), then heated to the desired temperature for 24 hours. The solvent was removed and the residue was purified by flash chromatography on silica gel using a mixture of methanol *in vacuo* (5 - 30 %) and methylene chloride, and/or recrystallization with methanol or isopropanol. The spectroscopic data for the coupling products follow.

### Compound 4: 2',3',5'-Triacetyl-8-N-morpholine-adenosine carboxyamide

¹H NMR (DMSO/D₂O) δ 2.05 (s, 3H), 2.10 (s, 3H), 2.14 (s, 3H), 3.77 (m, 2H), 3.86 (m, 6H), 4.36 (m, 2H), 4.49 (m, 1H), 5.84 (t, *J =* 6.3 Hz, 1H), 5.92 (s, 2H), 6.12 (dd, *J₁* = 6.3, *J₂* = 4.0 Hz, 1H), 6.44 (d, *J* = 4.0 Hz, 1H), 8.40 (s, 1H). ¹³C NMR (DMSO/D₂O) δ 20.5, 20.5, 20.7, 42.8, 47.9, 63.1, 66.6, 66.9, 70.4, 73.3, 79.9, 88.1, 118.3, 142.9, 150.1, 152.2, 155.1, 158.7, 169.6, 169.7, 170.6. HRMS (FAB+) m/z 507.1838 (Calc. 507.1840 for C₂₁H₂₆N₆O₉+H⁺).

### Compound 5: 2',3',5'-Triacetyl-8-N-(4-methylpyridyl)-adenosine carboxyamide

¹H NMR (CDCl₃) δ 2.03 (s, 3H), 2.09 (s, 3H), 2.14 (s, 3H), 4.38 (m, 2H), 4.49 (m,1H), 4.63 (d, *J* = 6.2 Hz, 2H), 5.93 (s, 2H), 5.99 (t, *J* = 6.5 Hz, 1H), 6.25 (dd, *J₁* = 6.4, *J₂* = 3.6 Hz, 1H), 7.28 (d, *J* = 4.3 Hz, 2H), 7.40 (d, *J* = 3.4 Hz, 1H), 8.15 (t, *J* = 6.3 Hz, 1H), 8.36 (s, 1H), 8.58 (d, *J* = 4.8 Hz, 2H). ¹³C NMR (CDCl₃) δ 20.5, 20.7, 42.1, 63.3, 70.4, 73.2, 79.6, 88.0, 118.2, 122.2, 140.3, 146.5, 150.1, 151.0, 154.5, 156.2, 158.7, 169.6, 169.8, 170.1. HRMS (FAB+) m/z 528.1842 (Calc. 528.1843 for C₂₃H₂₅N₇O₈+H⁺).

### Compound 6: 8-N-(4-pyridylmethyl)-adenosine carboxyamide

¹H NMR (D₂O) d 3.72 (dd, *J₁* = 12.5, *J₂* = 2.3 Hz, 1H), 3.88 (dd, *J₁* = 10.5, *J₂* = 1.9 Hz, 1H), 4.16 (m, 1H), 4.37 (m, 1H), 4.65 (s, 2H), 4.98 (m, 1H), 7.14 (d, *J* = 7.1 Hz, 1H), 7.43 (d, *J* = 5.6 Hz, 2H), 8.18 (s, 1H), 8.47 (d, *J* = 5.7 Hz, 2H). ¹³C NMR (DMSO/D₂O) δ 43.0, 64.1, 73.0, 74.7, 88.5, 91.3, 120.1, 124.0, 143.2, 150.2, 150.6, 151.5, 154.6, 158.8, 161.0. HRMS (FAB+) m/z 402.1522 (Calc. 402.1526 for C₁₇H₁₉N₇O₅+H⁺).

### Compound 7: 8-N-(n-Butyl)-adenosine carboxyamide

¹H NMR (DMSO) δ 0.91 (t, *J* = 7.2 Hz, 3H), 1.34 (m, 2H), 1.52 (m, 2H), 3.31 (t, *J* = 7.2 Hz, 1H), 3.53 (m, 1H), 3.68 (m, 2H), 3.95 (d, *J* = 2.7 Hz, 1H), 4.20 (m, 1H), 4.96 (dd, *J₁* = 12.0, *J₂* = 6.4 Hz, 1H), 5.13 (d, *J* = 4.4 Hz, 1H), 5.25 (d, *J* =6.4 Hz, 1H), 5.59 (dd, *J₁* = 8.9, *J₂* = 3.4 Hz, 1H), 6.69 (d, *J* = 6.7 Hz, 1H), 7.60 (s, 2H), 8.18 (s, 1H), 8.73 (t, *J* = 5.8 Hz, 1H); ¹³C NMR (DMSO) δ 23.2, 29.1, 40.5, 48.1, 71.8, 80.4, 81.3, 95.8, 98.6, 127.4, 152.2, 159.5, 162.9, 166.4, 168.2. HRMS (FAB+) m/z 367.1723 (Calc. 367.1729 for C₁₅H₂₃N₆O₅+H⁺).

### Compound 8: 8-N-(2-Propyl)-adenosine carboxyamide

¹H NMR (CD₃OD) δ 1.27 (d, *J* = 6.5 Hz, 6H), 3.73 (dd, *J₁* = 12.5, *J₂* = 2.6 Hz, 1H), 3.89 (dd, *J₁* = 12.5, *J₂* = 2.2 Hz, 1H), 4.17 (m, 1H), 4.21 (q, *J* = 6.5 Hz, 1H), 4.37 (dd, *J₁* = 5.3, *J₂* = 1.9 Hz, 1H), 4.96 (dd, *J₁* = 6.9, *J₂* = 4.3 Hz, 1H), 7.07 (d, *J* = 7.2 Hz, 1H), 8.19 (s, 1H). ¹³C NMR (CD₃OD δ 22.5, 43.1, 64.1, 72.9, 74.7, 88.4, 91.3, 119.9, 144.0, 151.4, 154.4, 158.7, 159.8. HRMS (FAB) m/z 353.1574 (Calc. 353.1573 for C₁₄H₂₀N₆O₅+H⁺).

### Compound 9: 8-N-(t-Butyl)-adenosine carboxyamide

¹H NMR (DMSO/D₂O) δ 1.37 (s, 9H), 3.52 (dd, *J₁* = 12.3, *J₂* = 3.7 Hz, 1H), 3.66 (dd, *J₁* = 12.5, *J₂* = 3.1 Hz, 1H), 3.94 (m, 1H), 4.18 (m, 1H), 4.91 (t, J = 5.5 Hz, 1H), 6.74 (d, *J* = 6.8 Hz, 1H), 8.15 (s, 1H). ¹³C NMR (DMSO/D₂O) δ 28.7, 52.0, 62.7, 71.3, 72.4, 86.7, 89.5, 118.1, 143.2, 150.4, 153.9, 157.2, 158.6. HRMS (FAB+) m/z 367.1723 (Calc. 367.1717 for C₁₅H₂₂N₆O₅+H⁺).

### Compound 10: 8-N-Phenyl-adenosine carboxyamide

¹H NMR (DMSO/D₂0) δ 3.74 (dd, *J₁* = 12.5, *J₂* = 2.6 Hz, 1H), 3.91 (dd, *J₁* = 12.6, *J₂* = 2.3 Hz, 1H), 4.19 (m, 1H), 4.39 (dd, *J₁* = 5.4, *J₂* = 2.0 Hz, 1H), 4.99 (dd, *J₁=* 7. *1, J₂* = 5.5 Hz, 1H), 7.18 (m, 2H), 7.39 (t, *J* = 5.6 Hz, 2H), 7.74 (d, *J* = 5.6 Hz, 2H), 8.21 (s, 1H). ¹³C NMR (DMSO) δ 62.2, 70.9, 71.9, 86.4, 89.1, 118.0, 120.0, 124.5, 128.9, 138.0, 142.7, 150.1, 153.7, 157.0, 157.3. HRMS (FAB+) m/z 387.1419 (Calc. 387.1417 for C₁₇H₁₈N₆O₅+H⁺).

### Compound 11: 8-N-(2-(N',N',N'-trimethylammonium)ethyl)-adenosine

carboxyamide chloride. ¹H NMR (CD₃OD) δ 3.27 (s, 9H), 3.65 (t, *J* = 6.4 Hz, 2H), 3.73 (dd, *J₁* = 12.5, *J₂* = 2.5 Hz, 1H), 3.90 (m, 3H), 4.17 (m, 1H), 4.36 (dd, *J₁* = 5.3, *J₂* = 1.7 Hz, 1H), 4.96 (dd, *J₁* = 7.1, *J₂* = 5.4 Hz, 1H), 7.16 (d, *J* = 7.2 Hz, 1H), 8.19 (s, 1H). ¹³C NMR (CD₃OD) δ 35.0, 54.1, 64.1, 65.6, 73.0, 74.6, 88.7, 91.1, 120.1, 142.7, 151.5, 154.7, 158.9, 161.1. HRMS (FAB+) m/z 396.1995 (Calc. 396.1995 for C₁₆H₂₆N₇O₅-Cl⁻).

### Compound 12: 8-N-(2-hydroxyethyl)-adenosine carboxyamide

¹H NMR (DMSO/D₂O) δ 3.35 (m, 2H), 3.53 (m,3H), 3.67 (dd, *J₁* = 12.5, *J₂* = 3.1 Hz, 1H), 3.96 (m, 1H), 4.17 (m, 1H), 4.89 (t, *J* = 5.9 Hz, 1H),6.86 (d, *J* = 6.8 Hz, 1H), 8.15 (s, 1H). ¹³C NMR (DMSO/D₂O) δ 42.0, 59.8, 62.7, 71.3, 72.4, 86.7, 89.5, 118.0, 142.4, 150.5, 154.0, 157.3159.7. HRMS (FAB) m/z 355.1372 (Calc. 355.1366 for C₁₃H₁₈N₆O₆+H⁺).

### Compound 13: 8-N-(Arginine ethyl ester)-adenosine carboxyamide

¹H NMR (CD₃OD) δ 1.28 (t, *J* = 7.1 Hz, 3H), 1.75 (m, 2H), 1.96 (m, 1H), 2.07 (m, 1H), 3.27 (m, 2H), 3.73 (dd, *J₁ =* 12.6, *J₂* = 2.7 Hz, 1H), 3.88 (dd, *J₁* = 12.6, *J₂* = 2.3 Hz, 1H), 4.18 (m, 1H), 4.23 (q, *J* = 7.1 Hz, 2H), 4.41 (dd, *J₁* = 5.4, *J₂* = 1.9 Hz, 1H), 4.67 (dd, *J₁* = 8.9, *J₂* = 5.0 Hz, 1H), 5.01 (dd, *J₁* = 7.0, *J₂* = 5.5 Hz, 1H), 7.09 (d, *J* = 7.1 Hz, 1H), 8.17 (s, 1H). ¹³C NMR (D₂O/CD₃0D) δ 14.4, 25.6, 28.7, 41.5, 53.8, 63.1, 63.9, 72.0, 74.0, 87.5, 90.3, 119.2, 142.4, 150.4, 154.4, 157.4, 157.7, 160.1,174.0. HRMS (FAB+) m/z (Calc. 387.1417 for C₁₇H₁₈N₆O₅+H⁺).

### Compound 14: 8-N-(t-Butyl)-guanosine carboxyamide

¹H NMR (CD₃OD) δ 1.44 (s, 9H), 3.74 (dd, *J₁* = 12.2, *J₂* = 3.7 Hz, 1H), 3.86 (dd, *J₁* = 12.2, *J₂* = 2.9 Hz, 1H), 4.07 (m, 1H), 4.39 (dd, *J₁* = 5.8, *J₂* = 3.4 Hz, 1H), 4.95 (t, *J* = 6.0 Hz, 1H), 6.93 (d, *J* = 6.4 Hz, 1H). ¹³C NMR (DMSO/D₂O) δ 28.9, 52.9, 64.0, 72.4, 73.8, 87.4, 91.1, 117.5, 141.6, 154.1, 155.5, 159.7, 159.9. HRMS (FAB+) m/z 383.1676 (Calc. 383.1679 for C₁₅H₂₂N₆O₆+H⁺).

**Compound 15:** 8-*N*-(2-(*N'*,*N'*,*N*'-trimethylammonium)ethyl)guanosine carboxyamide chloride. ¹H NMR (D₂O) δ 3.24 (s, 9H), 3.64 (t, *J* = 6.6 Hz, 2H), 3.91 (m, 4H), 4.20 (m, 1H), 4.52 (dd, *J₁* = 5.1, *J₂* = 3.7 Hz, 1H), 5.05 (t, *J* = 6.0 Hz, 1H), 6.85 (d, *J* = 6.2 Hz, 1H); ¹³C NMR (CD₃OD) δ 34.6, 54.4, 63.0, 65.0, 71.7, 73.1, 86.6, 90.1, 117.6, 140.1, 153.9, 155.1, 160.5, 161.1. HRMS (FAB+) m/z 412.1950 (Calc. 412.1945 for C₁₆H₂₆N₇O₆-Cl⁻).

### Compound 16: 8-N-(Arginine ethyl ester)-guanosine carboxyamide

¹H NMR (CD₃OD) δ 1.28 (t, *J* = 7.1 Hz, 3H), 1.72 (m, 2H), 1.89 (m, 1H), 2.03 (m, 1H), 3.23 (m, 2H), 3.74 (dd, *J₁* = 12.1, *J₂* = 3.8 Hz, 1H), 3.86 (dd, *J₁* = 2.2, *J₂* = 2.9 Hz, 1H), 4.06 (m, 1H), 4.21 (q, *J* = 7.1 Hz, 2H), 4.39 (dd, *J₁* = 5.7, *J₂* = 2.4 Hz, 1H), 4.61 (dd, *J₁* = 9.1*, J₂* = 4.7 Hz, 1H), 4.98 (t, *J* = 6.0 Hz, 1H), 6.98 (d, *J* = 6.3 Hz, 1H). HRMS (FAB+) m/z 512.2219 (Calc. 512.2217 for C₁₉H₂₉N₉O₈+H⁺).

### Compound 17: 8-N-(4-pyridylmethyl)-guanosine carboxyamide

¹H NMR (DMSO/D₂O) δ 3.51 (m, 1H), 3.64 (dd, *J₁* = 11.9, *J₂* = 4.4 Hz, 1H), 3.79 (m, 1H), 4.17 (dd, *J₁* = 5.5, *J₂* = 4.3 Hz, 1H), 4.42 (d, *J* = 6.3 Hz, 2H), 4.91 (t, *J =* 5.8 Hz, 1H), 6.62 (s, 2H), 6.71 (d, *J* = 5.8 Hz, 1H), 7.29 (d, *J* = 5.7 Hz, 2H), 8.49 (d, *J* = 5.3 Hz, 2H), 9.45 (t, *J* = 6.2 Hz, 1H). ¹³C NMR (DMSO/D₂O) δ 41.3, 62.2, 70.4, 71.1, 85.4, 89.0, 116.3, 122.3, 138.3, 148.4, 149.6, 152.8, 153.7, 156.7, 159.1. HRMS (FAB+) m/z 418.1482 (Calc. 418.1488 for C₁₉H₂₁N₄O₇+H⁺).

### Compound 18: 8-N-(2-Aminoethyl biotinamide)-guanosine carboxyamide

¹H NMR (CD₃OD) δ 1.17 (m, 2H), 1.26 (t, *J =* 12.0 Hz, 2H), 1.39 (m, 1H), 1.50 (m, 3H), 2.23 (t, *J* = 12.5 Hz, 2H), 2.63 (d, *J* = 22 Hz, 1H), 2.80 (dd, *J₁* = 22, *J*₂ =8.0 Hz, 1H), 2.90 (m, 1H), 3.18 (m, 2H), 3.48 (m, 4H), 3.81 (dd, *J₁* = 19.5, *J₂* = 6.0 Hz, 1H), 3.91 (dd, *J₁* = 11.5, *J₂* = 4.0 Hz, 1H), 4.08 (m, 1H), 4.19 (m, 1H), 4.40 (m, 1H), 4.47 (m, 1H), 4.96 (t, *J* = 10 Hz, 1H), 6.83 (d, *J* = 11 Hz, 1H). HRMS (FAB+) m/z 596.2251 (Calc. 596.2264 for C₂₃H₃₃N₉O₈S⁺H⁺).

### Example 2

### Purine Modification with Alcohols

The general procedure outline in Example I was followed to produce the modified purine described by the following scheme with the following results. 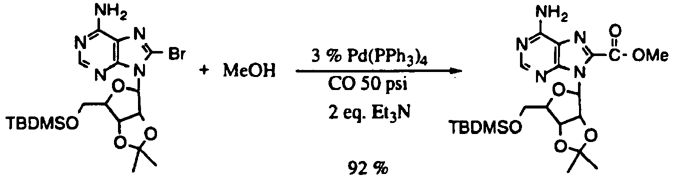 ¹H NMR (DMSO/D₂O) δ -0.05 (s, 3H), -0.04 (s, 3H), 0.82 (s, 9H), 1.38 (s, 3H), 1.60 (s, 3H), 3.67 (dd, J=10.5, 6.5 Hz, 1H), 3.78 (dd, J=10.6, 6.5 Hz, 1H), 4.03 (s, 3H), 4.25 (m, 1H), 5.10 (dd, J=6.5, 3.8 Hz, 1H), 5.69 (dd, J=6.4, 2.1 Hz, 1H), 6.34 (s, 2H), 7.04 (d, J=2.2 Hz, 1H), 8.36 (s, 1H).

### Example 3

### Uridine Modifications with Amines and Alcohols

The following procedures were employed to produce the modified uridine nucleosides described in Table II. 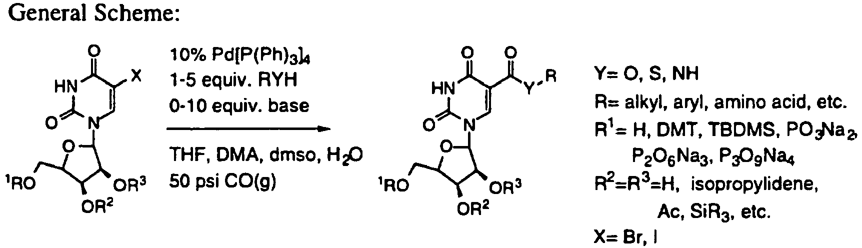 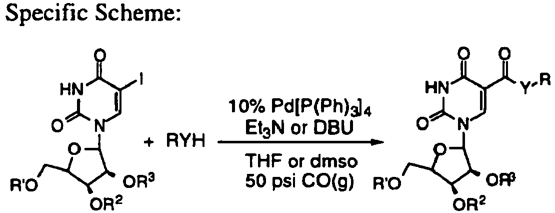 19, R¹ = H; R²,R³=isopropylidene
20, R¹ =DMT; R²,R³=isopropylidene
21, R¹ =TBDMS; R²,R³=isopropylidene
22, R¹ =PO₃Na; R²=R³=H

| **RYH** | | | | | | | |
|---|---|---|---|---|---|---|---|
| A | 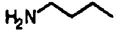 | F | 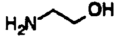 | J | 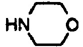 | N | 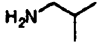 |
| B | 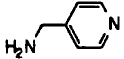 | G | 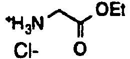 | K | 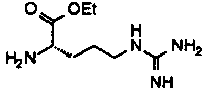 | O | 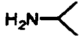 |
| C | 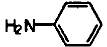 | H | 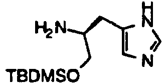 | L | 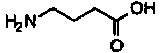 | P | 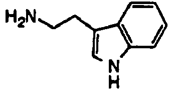 |
| D | 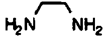 | | | | | | |
| E | HO-CH₃ | I | 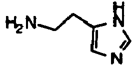 | M | 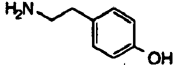 | Q | 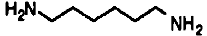 |

**Table II. Summary of uridine carboxyamidation products.**

| Entry | Nucleoside starting material | RYH | Product ID | Isolated Yield (%) |
|---|---|---|---|---|
| 1 | **19** | A | **23** | 65 |
| 2 | **19** | B | **24** | 89 |
| 3 | **19** | C | **25** | 20 |
| 4 | **19** | D | **26** | 78 |
| 5 | **20** | E | **27** | <20 |
| 6 | **21** | E | **28** | <20 |
| 7 | **20** | H | **29** | 69 |
| 8 | **21** | I | **30** | 68 |
| 9 | **21** | F | **31** | 61 |
| 10 | **21** | G | **32** | 80 |
| 11 | **21** | J | **33** | 68 |
| 12 | **21** | K | **34** | 57 |
| 13 | **21** | P | **35** | 43 |
| 14 | **21** | Q | **36** | 62 |
| 15 | **22** | B | **37** | 90 |
| 16 | **22** | L | **38** | 74 |
| 17 | **22** | M | **39** | 66 |
| 18 | **22** | N | **40** | 96 |
| 19 | **22** | O | **41** | 90 |
| 20 | **22** | A | **42** | 86 |
| 21 | **22** | P | **43** | 34 |

### Starting Material Syntheses.

Compound **22** was prepared according to a literature procedure (P.K. Chang and A.D. Welch, J. Med. Chem. 1963, 6, 428). The other starting materials (Compounds 19-21) were synthesized by the following procedures.

**Compound 19. 5.iodo.2'.3"O.isopropylideneuridine**. To a strirred solution of 5.00 g of 5-iodouridine (13.5 mmol) in 300 mL of acetone was added 250 mg of p-toluenesulfonic acid (1.30 mmol). The flask was fitted with an addition funnel filled with 4Å molecular sieves and a reflux condenser. The solution was heated at reflux temperature for 2h., after which all solids had dissolved. The flask was allowed to cool to room temperature and the solution concentrated *in vacuo.* The solution was dissolved in acetone, filtered through a plug of silica and the filtrate concentrated to give a pale yellow solid. This material was re-crystallized from ethanol to give the product as white needles in quantitative yield.

**Compound 20. 5'-DMT-5-iodo-2'.3'-O-isopropylideneuridine**. To a stirred solution of 820 mg of 5-iodo-2',3'-isopropylideneuridine (2.00 mmol) in 1.0 mL of anhydrous DMF and 1.8 mL of anhydrous pyridine, under argon, was added 24.4 mg of 4-dimethylaminopyridine (0.20 mmol) and 745 mg of DMTCI (2.20 mmol). The solution was stirred at room temperature overnight, diluted with 150 mL of ethyl acetate, washed with 3x75 mL of H₂O, 1 x50 mL of brine, and concentrated *in vacuo.* The residue was purified on silica gel with 40% EtOAc/hexanes to give 1.28 g (90% yield) of the product as a white solid.

**Compound 21. 5'-TBDMS-5-iodo-2'.3'-O-isopropylideneuridine**. To a stirred solution of 1.00 g of 5-iodo-2',3'-isopropylideneuridine (2.40 mmol) in 1.9 mL of anhydrous pyridine was added 724 mg of TBDMSCl (4.80 mmol). The solution was stirred overnight at room temperature, diluted with 30 mL of ethyl acetate and washed with 3x20 mL of H₂O, 1x 20 mL of brine and concentrated *in vacuo.* The residue was purified on silica gel with 30% EtOAc/hexanes to give 1.15 g (91 % yield) of the product as a white solid.

### Modified Uridine Syntheses

The modified uridines described in Table II were synthesized as follows.

**Compound 23. 5-(N-Butylcarboxyamidel-2'.3'-*O*-isopropylideneuridine.** To a 300 mL stainless steel Parr bomb in an argon atmosphere glove box was added a solution of 5-iodo-2',3'-*O*-isopropylideneuridine (0.351 g, 1.00 mmol, in 3.0 mL of THF), 10 mL of 1.0 M Et₃N/THF (10 mmol), 3.0 mL of 1.0 M n-butylamine in THF (3.0 mmol), and tetrakis(triphenyl-phosphine)palladium (0.116 g, 0.100 mmol). The bomb was sealed, removed from the box, evacuated and charged three times with 100 psi CO, then heated at 70 °C for 24h. The bomb was allowed to cool to room temperature, vented carefully in a fume hood, and the volatiles removed *in vacuo.* The crude reaction material was purified on silica gel with 5% MeOH/CH₂Cl₂ to give the product as a yellow solid (0.251 g, 65% yield). Analytical samples were obtained by crystallization from MeOH to give the product as fluffy white needles. ¹H NMR (dmso-d₆) δ 11.92 (br s, 1H), 8.68 (t, *J* = 5.4 Hz, 1H), 8.61 (s, 1H), 5.85 (d, *J* = 1.8 Hz, 1H), 5.09 (t, *J* = 4.5 Hz, 1H), 4.91 (dd, *J₁* = 6.3, *J₂* = 1.8 Hz, 1H), 4.74 (dd, *J₁* = 6.0, **J₂** = 2.7 Hz, 1H), 4.19 (m, 1H), 3.56 (m, 2H), 3.24 (m, 2H), 1.47 (s, 3H), 1.4 (m, 2H), 1.3 (m, 2H), 1.27 (s, 3H), 0.9 (t, *J* = 7.2 Hz, 3H); ¹³C NMR (dmso-d₆) δ 163.3 (C4), 161.3 (CONHBu), 149.4 (C2), 146.9 (C6), 112.5 (CMe₂), 105.0 (C5), 92.8 (C1'), 87.4 (C4'), 84.4 (C2'), 80.7 (C3'), 61.2 (C5'), 38.0 (CONHCH₂-), 31.2 ,(NHCH₂CH₂-), 26.9 (CCH₃), 25.0 (CCH₃), 19.5 (NHCH₂CH₂CH₂CH₃), 13.6 (NHCH₂CH₂CH₂CH₃); HRMS: Calculated(observed) for C₁₇H₂₆N₃O₇: 384.1771(384.1772). Anal. calcd.(found) for C₁₇H₂₅N₃O₇: C, 53.26(53.46); H, 6.57(6.53); N, 10.96(10.98).

**Compound 24.** 5-[*N*-(4-pyridylmethyl)carboxyamidel-2',3'-*O-***isopropylideneuridine**. To a heavy-walled glass bomb was added 224 mg 2',3'-isopropylidene-5-iodouridine (0.542 mmol), 63 mg tetrakis(triphenylphosphine)palladium (0.0542 mmol) and anhydrous pyridine until the solids were dissolved. Pyridine was then removed *in vacuo* and the solids dried under high vacuum overnight. To the bomb was then added, under argon, 4 mL anhydrous THF, 0.75 mL triethylamine (5.42 mmol) and 0.22 mL 4-aminomethylpyridine (2.17 mmol). The bomb was evacuated and charged three times with CO and heated to 70°C for 2.5 days. The bomb was allowed to cool to room temperature, the solvent removed *in vacuo* and the crude material loaded onto a pad of silica with dichloromethane. The pad was eluted with dichloromethane, then the desired product eluted with 10% MeOH/CH₂Cl₂ and concentrated *in vacuo* to a pale yellow solid. This material was purified by flash chromatography on silica gel with 5% MeOH/CH₂Cl₂ to give 201 mg (89% yield) of the product as a pale yellow solid. This material was recrystallized from methanol to give analytical samples of pure product as white needles. ¹H NMR (dmso-d₆) δ 11.98 (s, 1H), 9.19 (t, J=6.3 Hz, 1H), 8.66 (s, 1H), 8.48 (d, 2H, J=4.5 Hz), 7.25 (d, J=5.7 Hz, 2H), 5.86 (d, J= 2.2 Hz, 1H), 5.10 (t, J=4.8 Hz, 1H), 4.93 (dd, J= 6.2, 2.2 Hz, 1H), 4.73 (dd, J= 6.3, 3.0 Hz, 1H), 4.49 (d, J=6.3 Hz, 2H), 4.20 (m, 1H), 3.56 (t, J=4.5 Hz, 2H), 1.47 (s, 3H), 1.27 (s, 3H). ¹³C NMR (dmso-d₆) δ 163.2 (C4), 161.9 (CONH-), 149.5 (pyr o-C), 149.4 (C2), 148.4 (pyr *p*-C), 147.4 (C6), 122.1 (pyr *m*-C), 112.6 (CMe₂), 104.8 (C5), 92.9 (C1'), 87.4 (C4'), 84.4 (C2'), 80.7 (C3'), 61.2 (C5'), 41.2 (NHCH₂-), 26.9 (CCH₃), 25.0 (CCH₃); HRMS: Calculated(observed) for C₁₉H₂₃N₄O₇: 419.1567(419.1569). UV spectrum: λₘₐₓ at 276 nm (e=13730 M⁻¹cm⁻¹).

**5-[*N*-(4-pyridylmethyl)carboxamide]-5'-triphosphate-uridine**. The 5'-hydroxyl compound prepared as described was converted to the 5'-triphosphate using a modified procedure of Ludwig and Eckstein, *J. Org. Chem.* **1989**, *54*, 631-635. After removal of the 2',3'-*O*-isopropylidene protecting group by stirring in H₂O with Dowex H⁺ 50W x 80 at 70°C, the crude triphosphate was purified successively on DEAE sephadex anion exchange resin and C18 RP-HPLC using 100 mM Et₃NH⁺ HCO₃⁻ and CH₃CN as the mobile phases. The purity of the compound was checked by analytical C 18 RP-HPLC, ¹H and ³¹P NMR (D₂O), and quantitated by its UV absorbance at 276 nm (ε=13700 M⁻¹cm⁻¹).

**Compound 25. 5-(*N*-phenylcarboxyamide)-2',3'-*O-*isopropylideneuridine**. To a heavy-walled glass bomb in an argon atmosphere glove box was added 2',3'-isopropylidene-5-iodouridine (0.261 g, 0.636 mmol), tetrakis(triphenylphosphine)palladium (0.083 g, 0.072 mmol), and 4.5 mL of 1.0 M Et₃N/THF (4.5 mmol). The bomb was sealed, removed from the box, and 0.3 mL of aniline added via syringe under argon. The flask was evacuated and charged three times with 50 psi CO and heated to 70 °C for 2 days. The bomb was cooled to room temperature, concentrated *in vacuo* and purified by flash chromatography on silica gel with 4-6.5% MeOH·NH₃/CH₂Cl₂ to give a slightly yellow solid. This material was recrystrallized from methanol to give 52 mg (20% yield) of the pure product as fine white needles. ¹H NMR (dmso-d₆) δ 12.16 (br s, 1H), 10.88 (s, 1H), 8.79 (s, 1H), 7.63 (d, *J* = 7.8 Hz, 2H), 7.34 (m, 2H), 7.09 (t, *J* = 7.4 Hz, 1H), 5.88 (d, *J* = 2.1 Hz, 1H), 5.16 (t, *J* = 4.7 Hz, 1H), 4.95 (dd, *J₁* = 6.3, *J₂*= 2.1 Hz, 1H), 4.76 (dd, *J₁* = 6.3, *J₂* = 2.7 Hz, 1H), 4.25 (m, 1H), 3.59 (m, 2H), 1.48 (s, 3H), 1.29 (s, 3H); ¹³C NMR (dmso-d₆) δ 163.6 (C4), 159.9 (CONH-), 149.3 (C2), 147.8 (C6), 138.1 (CONHC<), 129.0 (phenyl *m*-C), 124.0 (phenyl *p*-C), 119.5 (phenyl o-C), 112.5 (CMe₂), 104.6 (C5), 93.2 (C1'), 87.6 (C4'), 84.5 (C2'), 80.7 (C3'), 61.2 (C5'), 26.9 (CCH₃), 25.0 (CCH₃). HRMS: Calculated(observed) for C₁₉H₂₂N₃O₇: 404.1458(404.1468).

**Compound 26. 5'-TBDMS-5-(*N*-[2-(*N'*-trifluoroacetamido)ethyl]carboxyamide)-2',3'-D-isopropylideneuridine**. To a heavy-walled glass bomb in an argon atmosphere glove box was added 5'-TBDMS-5-iodo-2',3'-*O-*isopropylideneuridine (0.531 g, 1.01 mmol), tetrakis(triphenylphosphine)palladium (0.350 g, 0.303 mmol), Et₃N (0.704 mL, 5.05 mmol) and 2 mL of dry THF. The bomb was sealed, removed from the box and 0.203 mL ethylenediamine (3.03 mmol) added under positive argon flow. The bomb was sealed under argon, evacuated and charged three times with 50 psi CO and heated to 70 °C overnight. The bomb was allowed to cool to room temperature, vented slowly, the solvent removed *in vacuo* and the crude material purified on silica gel with 25% MeOH·NH₃/EtOAc to give 381 mg (78% yield) of the product as a white solid. This material was protected as the N-triflouroacetamide in the following manner. To a stirred solution of 381.0 mg of the above product (0.78 mmol) in 7.0 mL of anhyd. CH₂Cl₂ at 0°C was added dry pyridine (0.126 mL, 1.6 mmol) and (CF₃CO)₂O (0.13 mL, 0.94 mmol). The solution was stirred at 0 °C for 30 min. then 0.19 mL of (CF₃CO)₂O (1.33 mmol) and 0.13 mL of pyridine (1.7 mmol) was added. After 30 min. the reaction was allowed to warm to room temperature, concentrated *in vacuo* and purified by flash silica gel chromatography with 40% EtOAc/hexanes to give 174 mg (38% yield, 30% yield from iodouridine starting material) of the product as a white solid. ¹H NMR (dmso-d₆) δ 11.95 (s, 1H), 9.48 (t, *J* = 5.0 Hz, 1H), 8.81 (t, *J* = 5.8 Hz, 1H), 8.49 (s, 1H), 5.75 (d, *J* = 1.6 Hz, 1H), 4.89 (dd, *J₁* = 6.1, *J₂* = 1.7 Hz, 1H), 4.67 (dd, *J₁* = 6.1, *J₂* = 2.2 Hz, 1H), 4.36 (m, 1H), 3.77 (m, 2H), 3.4 (m, 2H), 3.3 (m, 2H), 1.48 (s, 3H), 1.29 (s, 3H), 0.78 (s, 9H), 0.00 (s, 3H), -0.04 (s, 3H); ¹³C NMR (dmso-d₆) δ 163.1 (C4), 162.0 (CONH-), 149.4 (C2), 147.0 (C5), 117.7, 113.9, 112.2, 104.4, 94.7, 87.8, 85.0, 81.0, 63.4, 38.1, 37.2, 26.8, 25.6, 24.9, 17.8, -5.8, -5.8. HRMS: Calculated(observed) for C₂₃H₃₆F₃N₄O₈Si: 581.2254(581.2249).

**5'-Triphosphate-5-[*N*-(2-aminoethyl)carboxyamide]-uridine**. The 5'-TBDMS protected ethylenediamine amide of uridine (prepared above) was desilylated with Et₃NH⁺F⁻ in CH₃CN for 2 days and purified on silica gel with 20% MeOH/CH₂Cl₂ to give the 5'-hydroxyl compound, as identified by ¹H and ¹³C NMR, and FAB⁺ mass spectrometry. This compound was used for the preparation of the 5'-triphosphate using a modified of procedure of Ludwig and Eckstein, *J. Org. Chem.* **1989**, *54*, 631-635. After removal of the 2',3'-O̅-isopropylidene protecting group by stirring in H₂O with Dowex H⁺ 50W x 80 at 70 °C, the product was purified on C18 RP-HPLC with 0.05 M TBK/CH₃CN as the mobile phase to give desired product in 9% yield. The product was characterized by ¹H and ³¹P NMR and FAB⁺ MS.

**Compound 27. 5'-DMT-5-carbomethoxy-2',3'-*O*-isopropylideneuridine**. In a glove box 5'-DMT-5-iodo-2',3'-*O*-isopropylideneuridine (1.0 mL of a solution of 0.10 g/mL, 0.14 mmol) was added to a small heavy-walled glass bomb. Solid tetrakis(triphenylphosphine)-palladium (16 mg, 0.014 mmol) was added, followed by 0.70 mmol of Et₃N as a 1.0 M solution in THF, and 3.0 mL of anhydrous methanol (distilled *in vacuo* over Mg). The bomb was evacuated and refilled with 50 psi of CO (3x), then sealed and heated to 70 °C with stirring for 3 days. The vessel was vented and the solvents removed in *vacuo,* and the residue dissolved in the minimum 5% MeOH/CH₂Cl₂, loaded onto a pad of silica gel, and eluted successively with CH₂Cl₂ (discarded) and 5% MeOH/CH₂Cl₂. The resultant material was purified on silica gel with 5% MeOH/CH₂Cl₂ to give the product as a colorless solid. ¹H NMR (CD₃OD) δ 8.67 (s, 1H), 7.6-6.8 (m, 13H), 5.83 (d, *J* = 2.1 Hz, 1H), 4.98 (dd, *J₁* = 6.2, *J₂* = 2.1 Hz, 1H), 4.59 (m, 1H), 4.32 (m, 1H), 3.76 (two s, total 6H), 3.41 (s, 3H), 3.36 (m, 2H), 1.51 (s, 3H), 1.29 (s, 3H). FAB⁺ m/z 667 (M+Na⁺), 645 (M+H⁺), 303 (DMT⁺).

**Compound 28. 5'-TBDMS-5-carbomethoxy-2'.3'-*O-*isopropylideneuridine.** This compound was prepared as described above for the 5'-DMT protected compound, except using 5'-TBDMS-5-iodo-2',3'-*O-*isopropylideneuridine as the starting material. The product was isolated by flash chromatography on silica gel as a colorless solid. ¹H NMR (CD₃OD) δ 8.58 (s, 1H), 5.71 (d, *J* = 2.1 Hz, 1H)), 4.89 (dd, *J₁* = 6.2, *J₂* = 2.2, 1H), 4.75 (dd, *J₁* = 6.0, *J₂* = 1.8 Hz), 4.49 (m, 1H), 3.88 (m, 2H), 3.78 (s, 3H), 1.53 (s, 3H), 1.34 (s, 3H), 0.83 (s, 9H), 0.05 (s, 3H), -0.01 (s, 3H). ¹³C NMR (CD₃OD) δ 164.9 (C4), 162.2 (COOMe), 151.3 (C2), 150.3 (C6), 114.4 (CMe₂), 104.4 (C5), 97.3 (C1'), 90.1 (C4'), 87.4 (C2'), 83.1 (C3'), 65.1 (C5'), 52.4 (OCH₃), 27.4 (CCH₃), 26.3 (SiC[CH₃]₃), 25.3 (CCH₃), 19.2 (SiC[CH₃]₃), -5.4 (SiCH₃), -5.5 (SiCH₃). HRMS: Calculated(observed) mass for C₂₀H₃₃N₂O₈Si: 457.2006(457.2006).

**Compound 29. 5'TBDMS-5-(*N*-histidinolcarboxyamide)-2',3'-*O-*isopropylideneuridine**. To a heavy-walled glass bomb in an argon atmosphere glove box was added 3.5 mL of a 100 mg/mL solution of 5'-TBDMS-5-iodo-2',3'-*O-*isopropylideneuridine (0.491 mmol), 57 mg tetrakis(triphenylphosphine)palladium (0.0491 mmol), 0.2 mL of triethylamine (1.473 mmol) and 0.5 mL of THF. The bomb was sealed, removed from the box and under argon 1.9 mL of a 100 mg/mL solution of TBDMS protected histidinol (0.736 mmol) was added. The bomb was sealed under argon, evacuated and charged three times with 50 psi CO, and heated at 70 °C for 48 h. The bomb was allowed to cool to room temperature, vented and the solvent removed *in vacuo.* The crude material was purified by chromatography on silica gel with either a gradient of 5-7% or 0-5% MeOH/CH₂Cl₂ to give 0.294 g (69% yield) of the desired product as a white solid. ¹H NMR (dmso-d₆) δ 11.9 (br s, 2H), 8.9 (d, *J* = 8.3 Hz, 1H), 8.6 (s, 1H), 7.5 (s, 1H), 7.3 (m, 9H), 6.8 (m, 4H), 6.7 (s, 1H), 5.9 (d, *J* = 1.3 Hz, 1H), 5.0 (dd, *J₁* = 6.3, *J₂* = 1.4 Hz, 1H), 4.5 (unres. dd, 1H), 4.2 (m, 2H), 3.6 (m, 2H), 3.3 (m, 2H), 2.5 (m, 2H), 1.5 (s, 3H), 1.2 (s, 3H), 0.9 (s, 9H), 0.01 (s, 6H). ¹³C NMR (dmso-d₆) δ 163.2, 160.8, 158.0, 149.2, 148.0, 144.7, 135.3, 135.2, 134.7, 129.7, 129.5, 127.7, 127.5, 126.6, 113.1, 112.9, 105.2, 93.8, 86.4, 85.7, 83.9, 80.7, 63.9, 63.1, 54.9, 50.1, 28.5, 26.8, 25.7, 25.0, 17.9, -5.6, -5.6. HRMS: Calculated(observed) mass for C₄₆H₅₇N₅O₁₀Si: 867.3874(867.3884).

**Compound 30. 5'-TBDMS-5-[*N*-(2-[4-imidazole]ethyl)carboxyamide]-2',3'-*O*-isopropylideneuridine**. To a heavy-walled glass bomb in an argon atmosphere glove box was added 5'-TBDMS-5-iodo-2',3'-O-isopropylideneuridine (0.260 g, 0.496 mmol), 4 mL of dry THF, and tetrakis(triphenylphosphine)palladium (0.073 g, 0.063 mmol). The bomb was sealed, removed from the box and the solvent removed *in vacuo.* Under argon, anhydrous Et₃N (0.35 mL, 2.48 mmol), histamine (0.263 g, 2.37 mmol) and 2 mL of dmso-d₆ were added. The bomb was evacuated and charged three times with 50 psi CO and heated at 70 °C for 2 days. After cooling to room temperature, the bomb was vented *carefully* and the solvents removed *in vacuo* at 70 °C. The crude material was purified by flash chromatography on silica gel with 12% MeOH/CH₂Cl₂ to give 181 mg (68% yield) as a slightly yellow solid. ¹H NMR (CD₃OD) δ 8.6 (s, 1H), 7.6 (s, 1H), 6.9 (s, 1H), 5.7 (d, *J* = 1.9 Hz, 1H), 4.7 (dd, *J₁* = 5.9, *J₂* = 1.6 Hz, 1H), 4.5 (m, 1H), 3.9 (m, 2H), 3.6 (m, 2H), 2.8 (t, 2H), 1.5 (s, 1H), 1.3 (s, 1H), 0.8 (s, 9H), 0.04 (s, 3H), -0.01 (s, 3H); ¹³C NMR (dmso-d₆) δ 163.2, 161.3, 149.4, 146.7, 134.7, 112.2, 111.8, 104.6, 94.6. 87.8, 84.9, 81.0, 63.4, 48.5, 27.0, 26.8, 25.6, 24.9, 17.9, -5.7; HRMS: Calculated(observed) for C₂₄H₃₇N₅O₇Si: 535.2462(535.2456). UV spectrum: λₘₐₓ at 278 nm (ε=12930 M⁻¹cm⁻¹).

**5'-Triphosphate-5-[*N*-(2-[4-imidazole]ethyl)carboxyamide]-uridine**. The 5'-TBDMS protected histamine amide of uridine (prepared above) was desilylated with Et₃NH⁺F- in CH₃CN for 2 days and purified on silica gel with 15% NH₃-MeOH/CH₂Cl₂ to give the 5'-hydroxyl histamine amide of uridine, as identified by ¹H and ¹³C NMR, and FAB⁺ mass spectrometry. This compound was used for the preparation of the 5'-triphosphate using a modified procedure of Ludwig and Eckstein, J. Org. Chem. 1989, 54, 631-635. After removal of the 2',3'-O-isopropylidene protecting group with acidic Dowex resin in H₂O at 70 °C, the crude triphosphate was purified successively on DEAE sephadex anion exchange resin and C18 RP-HPLC using 100 mM aq. Et₃NH⁺HCO₃⁻ and CH₃CN as the mobile phases. The purity of the compound was checked by analytical C 18 RP-HPLC, ¹H and ³¹P NMR (D₂O), and quantitated by its UV absorbance at 278 nm (using the ε for the nucleoside starting material, ε₂₇₈=12930 M⁻¹cm⁻¹).

**Compound 31. 5'-TBDMS-5-[*N*-(2-hydroxyethyl)carboxyamide]-2',3'-*O*-isopropylideneuridine**. This compound was prepared as described above for compound **30**, using 3 eq. of ethanolamine and 3 eq. of triethylamine, and allowed to react for 48h at 70 °C. The product was purified on silica gel with 6% MeOH/CH₂Cl₂ to give 0.173 g (61 % yield) of colorless white solid. ¹H NMR (dmso-d₆) δ 11.93 (s, 1H), 8.80 (t, *J* = 5.6 Hz, 1H), 8.48 (s, 1H), 5.75 (d, *J* = 1.8 Hz, 1H), 4.89 (dd, *J₁* = 6.1, *J₂* = 1.8 Hz, 1H), 4.78 (t, *J* = 5.1 Hz, 1H), 4.67 (unres. dd, 1H), 4.34 (m, 1H), 3.76 (d, *J* = 3.8 Hz, 2H), 3.44 (m, 2H), 3.31 (m, 2H), 1.47 (s, 3H), 1.28 (s, 3H), 0.78 (2, 9H), -0.02 (s, 3H), -0.04 (s, 3H). ¹³C NMR (dmso-d₆) δ 163.2 , 161.5, 149.4, 146.8, 112.3, 104.6, 94.5, 87.7, 84.9, 80.9, 63.4, 59.7, 41.2, 26.8, 25.6, 24.9, 17.9, -5.7, -5.8. Analytical sample from EtOAc/Hexanes. FAB⁺ HRMS calculated(observed) for C₂₁H₃₆N₃O₈Si: 486.2272(486.2271). Anal. Calcd. (Found) for C₂₁H₃₅N₃O₈Si: C, 51.94 (52.03); H, 7.26 (7.36); N, 8.65 (8.61).

**Compound 32. 5'-TBDMS-5-[*N*-((2-carboethoxl)ethyl)carboxyamide]-2',3'-*O*-isopropylideneuridine**. This compound was prepared as described above for compound 30, using 1.0 eq. of glycine ethyl ester hydrochloride and 3 eq. of triethylamine. The product was purified on silica gel with 4% MeOH/CH₂Cl₂ to give 0.262 g (80% yield) of colorless white solid. ¹H NMR (CDCl₃) δ 8.96 (t, *J* = 5.5 Hz, 1H), 8.72 (s, 1H), 8.65 (s, 1H), 5.74 (d, *J* = 2.2 Hz, 1H), 4.85 (dd, *J₁* = 6.0, *J₂* = 2.2 Hz, 1H), 4.72 (dd, *J₁* = 6.0, *J₂* = 1.6 Hz, 1H), 4.51 (m, 1H), 4.22 (q, *J* = 7.1, 2H), 4.14 (d, *J* = 5.6 Hz, 2H), 3.96 (m, 1H), 3.78 (m, 1H), 1.58 (s, 3H), 1.36 (s, 3H), 1.28 (t, *J* = 7.1 Hz, 3H), 0.82 (s, 9H), 0.04 (s, 3H), -0.01 (s, 3H). ¹³C NMR (CDCl₃) δ 169.6, 163.1, 161.8, 149.4, 147.3, 112.3, 104.1, 94.6, 87.8, 84.9, 81.0, 63.4, 60.4, 40.8, 26.9, 25.6, 24.9, 17.9, 14.0, -5.8. Analytical sample from EtOAc/Hexanes. FAB⁺ HRMS calculated(observed) for C₂₃H₃₈N₃O₉Si: 528.2377(528.2382). Anal. Calcd. (Found) for C₂₃H₃₇N₃O₉Si: C, 52.36 (52.19); H, 7.07 (6.93); N, 7.96 (7.85).

**Compound 33. 5'-TBDMS-5-[morpholinecarboxyamide]-2',3'-*O-*isopropylideneuridine**. This compound was prepared as described above for compound 30, using 3 eq. of morpholine and 3 eq. of triethylamine. The product was purified on silica gel with 4% MeOH/CH₂Cl₂ 0.202 g (68% yield) of colorless white solid. ¹H NMR (dmso-d₆) δ 11.65 (s, 1H), 7.90 (s, 1H), 5.80 (d, *J*=2.2 Hz, 1H), 4.93 (dd, *J*=6.2, 2.2 Hz, 1H), 4.68 (6.2, 3.4 Hz, 1H), 4.12 (m, 1H), 3.76 (m, 2H), 3.5 (br m, 6H), 3.29 (br m, 2H), 1.47 (s, 3H), 1.28 (s, 3H), 0.84 (s, 9H), 0.03 (s, 6H). ¹³C NMR (dmso-d₆) δ 162.3, 160.3, 149.7, 142.2, 112.9, 111.1, 66.2, 65.9, 63.0, 47.0, 41.9, 26.9, 25.8, 25.1, 18.0, -5.5, -5.6. Analytical sample from EtOAc/Hexanes. FAB⁺ HRMS calculated(observed) for C₂₃H₃₈N₃O₈Si: 512.2428(512.2436).

**Compound 34. 5'-TBDMS-5-[*N*-(arginine ethyl ester)carboxyamide]-2',3'-*O*-isopropylideneuridine**. To a heavy-walled glass bomb in an argon atmosphere glove box was added 5'-TBDMS-5-iodo-2',3'-*O*-isopropylideneuridine (0.238 g, 0.453 mmol), arginine ethyl ester dihydrochloride (0.260 g, 0.94 mmol), tetrakis(triphenylphosphine)-palladium (0.052 g, 0.045 mmol), Et₃N (0.32 mL, 2.3 mmol), 3 mL of dry THF, and 2 mL of DMSO. The reaction vessel was evacuated and charged with 50 psi of CO three times, then heated to 70 °C for 2 days. The crude mixture was concentrated and chromatographed on silica gel with 25% MeOH-NH₃/EtOAc to give the product as an off-white solid, 0.160 g (57% yield). ¹H NMR (CD₃OD) δ 8.55 (s, 1H), 5.64 (d, *J* = 1.9 Hz, 1H), 4.77 (unres. dd, 1H), 4.68 (unres dd, 1H), 4.22 (q, *J* = 7.1 Hz, 2H), 4.17 (m, 1H), 3.93 (m, 1H), 3.75 (m, 2H), 3.43 (br m, 2H), 2.0 (br m, 1H), 1.78 (br m, 3H), 1.50 (s, 3H), 1.34 (s, 3H), 1.26 (t, *J* = 7.1 Hz, 3H), 0.92 (s, 9H), 0.13 (s, 6H).

**Compound 35. 5-[*N*-(2-[3-indolyl]ethyl)carboxyamide)-2',3'-*O-*isopropylideneuridine**. To a heavy-walled glass bomb in an argon atmosphere glove box was added 5'-TBDMS-5-iodo-2',3'-*O*-isopropylideneuridine (1.13 g, 2.14 mmol), 2-(3-indolyl)ethylamine (1.70 g, 10.7 mmol) and tetrakis(triphenylphosphine)palladium (0.247 g, 0.214 mmol), anhydrous triethylamine (1.5 mL, 10.7 mmol), and 10 mL of THF. The bomb was sealed, and evacuated and charged three times with 50 psi CO, then heated at 70 °C for 16h. After cooling, and removal of the solvents *in vacuo*, the crude material was purified on silica gel with 0-5% MeOH/CH₂Cl₂ to give 0.895 g of slightly impure yellow solid (78% crude yield). Desilylation was accomplished by stirring the above material in 2 mL of anhyd. CH₃CN with Et₃N•HF (1.0 g, 8.4 mmol) for 16h at ambient temp. The reaction mixture was diluted with 30 mL of EtOAc and extracted with 3x20 mL of H₂O, 10 mL of brine, then concentrated *in vacuo* and purified on silica gel with 0-5% MeOH/CH₂Cl₂ to give the desired product as a pale yellow solid (0.430 g, 43% yield). ¹H MNR (CD₃OD) δ 10.2 (br s, 1H), 9.1 (t, 1H), 8.7 (s, 1H), 7.5 (d, *J* = 7.8 Hz, 1H), 7.3 (d, *J* = 8.1 Hz, 1H), 7.0 (m, 3H), 5.9 (d, *J* = 2.1 Hz, 1H), 4.3 (t, 1H), 3.7 (m, 4H), 3.0 (t, 2H), 1.5 (s, 1H), 1.3 (s, 1H).

**5'-Triphosphate-5-[N-(2-[3-indolyl]carboxyamide)uridine**. The 5'-TBDMS protected tryptamine amide of uridine (prepared above) was desilylated with 5 eq. of Et₃NH⁺F⁻ in CH₃CN for 18 h at RT and purified on silica gel to give the 5'-hydroxyl compound in 43% yield, as identified by ¹H and ¹³C NMR, and FAB⁺ mass spectrometry. The 5'-hydroxyl compound was then used for the preparation of the 5'-triphosphate using a modified of procedure of Ludwig and Eckstein, J. Org. Chem. 1989, 54, 631-635. After removal of the 2',3'-.*O*-isopropylidene protecting group by stirring in H₂O with Dowex H⁺ 50W x 80 at 70 °C, the product was purified on DEAE sephadex with 0.05-1.5 M TBK buffer with 25% added CH₃CN, followed by C18 RP-HPLC with 0.05 M TBK/CH₃CN mobile phase as eluant. The triphosphate was characterized by ¹H and ³¹P NMR and FAB⁺ MS.

**Compound 36. 5'-TBDMS-5-(N-[6-aminohexyl]-carboxyamide)-2'.3'-*O-*isopropylideneuridine**. To a heavy-walled glass bomb in an argon atmosphere glove box was added 5'-TBDMS-5-iodo-2',3'-*O*-isopropylideneuridine (1.28 g, 2.40 mmol), 1,6-diaminohexane (1.40 g, 12.0 mmol) and tetrakis(triphenylphosphine)palladium (0.83 g, 0.72 mmol) and 10 mL of THF. The bomb was sealed, removed from the box and triethylamine (1.7 mL, 12.0 mmol) was added under argon via syringe. The vessel was evacuated and charged three times with 50 psi CO and heated at 70 °C overnight. The bomb was allowed to cool, vented, and the solvent removed *in vacuo.* The crude material was dissolved in 10 mL of methanol and the palladium catalyst removed by filtration. The filtrate was concentrated and purified on silica gel with 15-25% NH₃-CH₃OH in CH₂Cl₂ to give the desired product (0.812 g, 62% yield) as a white solid. ¹H NMR (CD₃OD) δ 9.0 (s, 1H), 5.7 (d, *J* = 1.8 Hz, 1H), 4.9 (m, 1H), 4.7 (m, 1H), 4.5 (br s, 1H), 3.9 (m, 2H), 3.3 ( m, 3H), 2.9 (t, 2H), 1.5 (unres. m, 12H), 0.8 (s, 9H), 0.04 (s, 3H), 0.0 (s, 3H).

**5-(*N*-[6-*N*'-trifluoroacetamidohexyl]-carboxyamide)-2',3'-*O-*isopropylideneuridine**. The free amine of the above compound was protected in the following manner. To a stirred solution of compound **36** in acetonitrile with catalytic triethylamine was added 2.0 eq. ethyl triflouroacetate. The solution was stirred at RT for 18h, and an additional 1.6 eq. ethyl triflouroacetate added and the solution stirred for 20h. Concentration and purification on silica gel with 1-5% MeOH/CH₂Cl₂ gave the desired triflouroacetamide in 25% yield, as characterized by ¹H NMR. The compound was then desilylated with 5 eq. of Et₃NH⁺F⁻ in CH₃CN for 18 h at RT and purified on silica gel to give the 5'-hydroxyl compound in quantitative yield, as identified by its ¹H NMR spectrum.

**General prodedure for compounds 37-43**. To a glass pressure reactor equipped with a Teflon valve were added nucleotide (0.1 mmol), the amine (0.5 mmol, 5 eq.), Pd(PPh₃)₄ (0.01 mmol, 0.1 eq.), and dimethyl sulfoxide as solvent (0.5 mL). The reactor was evacuated and charged with CO (50 psi) three times before heating to 60 °C for 24 hours. The crude mixture was purified on a DEAE Sephadex A-25 anion exchange column using a linear gradient of 0.05 M to 1.0 M triethylammonium bicarbonate buffer. Characterization of the compounds follows.

**Compound 37**. ¹H NMR (D₂O) δ 4.0 (m, 2 H), 4.23 (m, 1 H), 4.28 (t, *J* = 4.5 Hz, 1 H), 4.43 (t, *J* = 5.2 Hz, 1 H), 4.60 (s, 2 H), 5.93 (d, *J* = 5.1 Hz, 1 H), 7.34 (d, *J* = 4.8 Hz, 2 H), 8.42 (d, *J* = 4.8 Hz, 2 H), 8.55 (s, 1 H). ¹³C NMR (CD₃OD) δ 43.2, 64.9, 71.1, 74.9, 85.1, 90.7, 106.9, 123.4, 147.7, 149.4, 150.2, 152.1, 165.0, 165.5. ³¹P NMR (CD₃OD) δ 6.4. HRMS (FAB+) m/z 459.0909 (Calc. 459.0917 for C₁₆H₁₉N₄O₁₀P+H⁺).

**Compound 38.** ¹H NMR (D₂O) δ 1.79 (m, 2 H), 2.20 (t, *J* = 7.5 Hz, 2 H), 3.34 (t, *J* = 6.9 Hz, 2 H), 4.08 (m, 2 H), 4.28 (m, 2 H), 4.37 (t, *J* = 4.8 Hz, 1 H), 5.92 (d, *J* = 4.9 Hz, 1 H), 8.55 (s, 1 H). ¹³C NMR (D₂O) δ 26.8, 36.0, 39.9, 65.0, 71.3, 74.6, 84.4, 90.6, 107.2, 146.2, 159.3, 167.1, 174.4, 183.6. ³¹P NMR (CD₃OD) δ 8.2. MS (FAB) m/z 454.0858 (Calc. 454.0863 for C₁₄H₂₀N₃O₁₂P+H⁺).

**Compound 39**. ¹H NMR (D₂O) δ 2.73 (t, *J* = 6.7 Hz, 2 H), 3.50 (t, *J* = 6.7 Hz, 2 H), 4.03 (m, 2 H), 4.25 (m, 2 H), 4.36 (t, *J* = 4.8 Hz, 1 H), 5.87 (d, *J* = 4.9 Hz, 1 H), 6.76 (d, *J* = 8.3 Hz, 2 H), 7.08 (d, *J* = 8.3 Hz, 2 H), 8.44 (s, 1 H). ¹³C NMR (CD₃OD) δ 34.6, 41.9, 65.0, 71.0, 74.9, 8439, 90.7, 106.9, 116.4, 131.2, 131.9, 147.2, 151.8, 155.0, 164.6, 164.7. ³¹P NMR (CD₃OD) δ 8.1. HRMS (FAB+) m/z 488.1082 (Calc. 488.1084 for C₁₈H₂₂N₃O₁₁P+H⁺).

**Compound 40**. ¹H NMR (D₂O) δ 0.87 (d, *J* = 6.7 Hz, 6 H), 1.82 (m, 1 H), 4.03 (m, 2 H), 4.27 (m, 2 H), 4.39 (t, *J* = 5.0 Hz, 1 H), 5.91 (d, *J* = 5.1 Hz, 1 H), 8.53 (s, 1 H). ¹³C NMR (MeOD) δ 20.3, 29.0, 43.3, 65.1, 71.0, 75.0, 85.0, 90.6, 107.2, 147.3, 151.9, 164.8, 165.0. ³¹P NMR (CD₃OD) δ 6.4. HRMS (FAB+) m/z 424.1129 (Calc. 424.1121 for C₁₄H₂₂N₃O₁₀P+H⁺).

**Compound 41.** ¹H NMR (D₂O) δ 1.16 (d, *J* = 5.4 Hz, 6 H), 4.02 (m, 3 H), 4.28 (m, 2 H), 4.38 (t, *J* = 5.0 Hz, 1 H), 5.90 (d, *J* = 3.6 Hz, 1 H), 8.50 (s, 1 H). ¹³C NMR (D₂O) δ 164.9, 163.7, 151.9, 147.1, 107.2, 90.6, 85.0, 75.0, 71.0, 65.1, 43.0, 22.5. ³¹P NMR (D₂O) δ 6.5.

**Compound 42.** ¹H NMR (D₂O) δ 0.86 (t, *J* = 7.3 Hz, 3 H), 1.32 (m, 2 H), 1.51 (m, 2 H), 3.31 (m, 2 H), 3.99 (m, 2 H), 4.23 (m, 1 H), 4.28 (m, 1 H), 4.40 (t, *J* = 5.1 Hz, 1 H), 5.91 (d, *J* = 5.1 Hz, 1 H), 8.50 (s, 1 H). ¹³C NMR (1:1 D₂O:CD₃OD) δ 14.0, 20.7, 31.8, 40.0, 65.0, 71.4, 75.0, 85.3, 90.5, 107.0, 147.2, 152.8, 164.8, 166.1. ³¹P NMR (D₂O) δ 7.7. HRMS (FAB+) m/z 424.1121 (Calc. 424.1121 for C₁₄H₂₂N₃O₁₀P+H⁺).

**Compound 43.** H NMR (D₂O) δ 2.88 (t, *J* = 6.3 Hz, 2H), 3.53 (t, *J* = 6.3 Hz, 2H), 3.96 (s, 2H), 4.20 (d, *J* = 2.3 Hz, 2H), 4.30 (t, *J* = 4.0 Hz, 1H), 5.78 (d, *J* = 4.7 Hz, 1H), 6.97 (t, *J* = 7.5 Hz, 1H), 7.08 (m, 2H), 7.34 (d, J = 8.0 Hz, 1H), 7.46 (d, J = 7.8 Hz, 1H), 8.22 (s, 1H). ¹³C NMR (D₂O) δ 25.0, 40.1, 64.8, 71.1, 74.7, 84.8, 90.8, 106.8, 112.7, 119.5, 120.0, 122.8, 124.5, 127.8, 137.2, 146.6, 152.7, 164.8, 165.9. ³¹P NMR (MeOD) δ 8.2. HRMS (FAB+) m/z 511.1236 (Calc. 511.1230 for C₂₀H₂₃N₄O₁₀P+H⁺).

### Example 4

### Cytidine Modifications with Amines

The following procedures were employed to produce the modified cytidines shown in Table III.

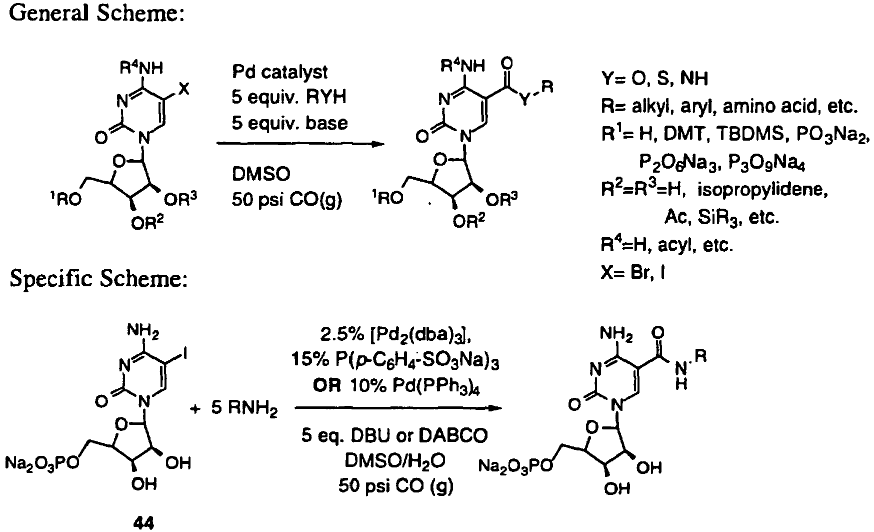

| **RYH** | | | | | | | |
|---|---|---|---|---|---|---|---|
| **A** | 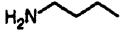 | **C** | 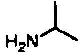 | **E** | 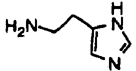 | **G** | 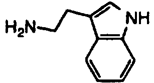 |
| **B** | 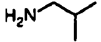 | **D** | 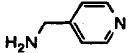 | **F** | 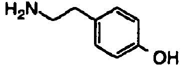 | | |

**Table III. Summary of cytidine carboxvamidation products.**

| Entry | Starting material | RNH₂ | Product ID | Isolated Yield (%) |
|---|---|---|---|---|
| 1^{a} | **44** | **A** | **45** | 73 |
| 2^{b} | **44** | **B** | **46** | 42 |
| 3^{a} | **44** | **C** | **47** | 80 |
| 4^{c} | **44** | **D** | **48** | 36 |
| 5^{c} | **44** | **E** | **49** | 20 |
| 6^{b} | **44** | **F** | **50** | 49 |
| 6^{c} | **44** | **G** | **51** | 81 |

| | | | | |
|---|---|---|---|---|
| ^{a} Nucleotide (0.1 mmol), RNH₂ (0.5 mmol), Pd(PPh₃)₄ (0.01 mmol), DBU (0.5 mmool), DMSO (0.5 mL). ^{b} Nucleotide (0.1 mmol), RNH₂ (0.5 mmol), Pd₂(dba)₃ (2.5 µmol), P(*p*-C₆H₄-SO₃Na)₃ (0.015 mmol), DABCO (0.7 mmol), DMSO (0.5 mL). ^{c} Nucleotide (0.1 mmol), RNH₂ (0.5 mmol), Pd(PPh₃)₄ (0.01 mmol), DBU or DABCO (0.5 mmol), DMSO:H₂O 93:7 (0.5 mL). | | | | |

### General Information

5-Iodocytidine monophosphate was synthesized according to a literature procedure (Voytek, P.; Chang, P.K.; Prusoff, W.H. *J. Biol. Chem.* 1971, *246,* 1432). Trisulfonated triphenylphosphine sodium salt was purchased from Strem Chemicals, Inc. All other compounds were purchased from Aldrich Chemical Co. n-Butylamine, isopropylamine, triethylamine, and 1,8-diazabicyclo(5.4.0)undec-7-ene (DBU) were purchased from Aldrich Chemical Co. and distilled from CaH₂. Isobutylamine, tyramine hydrochloride, and 4-aminobutyric acid (Acros Organics) were used as is. Histamine was purchased from Sigma Chemical Co. and used as is. Cytidine monophosphate, Pd₂(dba)₃, tetrakis(triphenylphosphine)palladium(0), DABCO, 4-aminomethylpyridine, and DMSO were purchased from Aldrich Chemical Co. and used as is. The ¹H and ¹³C NMR spectra were obtained in CD₃OD, D₂O, or DMSO-d⁶ using a Bruker ARX-300 spectrometer and referenced to solvent resonances. High resolution fast atom bomdardment mass spectra (HR FAB MS) were obtained using VG 70 SE & ZAB2-EQ/FAB(+).

### General procedure

The general procedure to synthesize the compounds of Table III is provided. To a heavy-walled glass pressure reactor equipped with a Teflon valve were added nucleotide (0.1 mmol), amine (0.5 mmol), Pd(PPh₃)₄ (0.01 mmol), DBU (0.5 mmol), DMSO (0.5 ml). The reactor was evacuated and charged with CO (50 psi) three times before heating to 60 °C for 24 hours. The crude mixture was quantitatively analyzed by reverse phase HPLC. The products were purified on a DEAE Sephadex A-25 ion exchange column using a linear gradient (0.05 M to 1.0 M) of triethylammonium bicarbonate followed by preparative C-18 reverse phase HPLC (0.05 M thiethylammonium hydrogen carbonate / MeOH). Characterization of the compounds listed in Table III follows.

**Compound 45. 5-(*N*-Butylcarboxyamide)-cytidine monophosphate.** ¹H NMR (CD₃OD) δ 0.85 (t, *J* = 7.4 Hz, 3H), 1.29 (m, 2H), 1.49 (m, 2H), 3.24 (m, 2H), 4.05 (m, 2H), 4.25 (m, 2H), 4.33 (m, 1H), 5.87 (d, *J* = 4.1 Hz, 1H), 8.38 (s, 1H). ¹³C NMR (CD₃OD) δ 14.2, 20.7, 31.7, 40.7, 64.6, 70.8, 75.6, 84.9. 91.1, 102.8, 144.3, 157.3, 164.8, 167.0. ³¹P NMR (CD₃OD) δ 5.8. HRMS (FAB+) m/z 423.1275 (Calc. 423.1281 for C₁₄H₂₃N₄O₉P+H⁺).

**Compound 46. 5-(*N*-Isobutylcarboxyamide)-cytidine monophosphate.** ¹H NMR (CD₃OD) δ 0.92 (d, *J* = 3.0 Hz, 3H), 0.94 (d, *J* = 3.0 Hz, 3H), 1.99 (m, 1H), 3.12 (m, 2H, overlapped with Et₃NH⁺), 4.07 (m, 1H), 4.16 (m, 2H), 4.29 (m, 2H), 5.99 (d, *J* = 4.9 Hz, 1H), 8.66 (s, 1H). ¹³C NMR (60:1 D₂O:CD₃OD) δ 20.6, 20.6, 29.0, 48.2, 64.8, 70.7, 75.5, 84.6, 91.4, 103.0, 144.3, 157.3, 164.8, 167.3.

**Compound 47. 5-(*N*-Isopropylcarboxyamide)-cytidine monophosphate.** ¹H NMR (CD₃OD) δ 1.22 (m, 6H), 4.06 (m, 3H), 4.24 (m, 1H), 4.28 (m, *J* = 4.9 Hz, 1H), 4.38 (t, *J* = 5.0 Hz, 1H), 5.89 (d, *J* = 4.8 Hz, 1H), 8.31 (s, 1H). ¹³C NMR (CD₃OD) δ 22.3, 43.5, 64.9, 70.8, 75.1, 84.8, 91.2, 103.3, 144.6, 157.4, 164.7, 166.5. ³¹P NMR (CD₃OD) δ 5.6. HRMS (FAB+) m/z 409.1119 (Calc. 409.1124 for C₁₃H₂₁N₄O₉P+H⁺).

**Compound 48. 5-[*N*-(4-pyridylmethyl)carboxyamide]cytidine monophosphate.** ¹H NMR (DMSO-d₆) δ 2.99 (q, *J* = 7.3 Hz, 6H), 3.88 (m, 1H), 3.98 (m, 2H), 4.03 (m, 1H), 4.13 (m, 1H), 4.32 (d, *J* = 5.2 Hz, 2H), 5.92 (d, *J* = 5.7 Hz, 1H), 7.32 (d, *J* = 5.4 Hz, 2H), 7.80 (s, 1H), 8.44 (d, *J* = 4.8 Hz, 2H), 8.50 (m, 1H), 8.79 (s, 1H), 10.38 (m, 1H). ¹³C NMR (DMSO-d₆) δ 41.8, 63.9, 70.4, 74.5, 83.7, 88.4, 98.6, 122.4, 144.0, 149.0, 149.2, 153.9, 162.3, 163.7, 165.2. ³¹P NMR (CD₃OD) δ 6.5. HRMS (FAB+) m/z 458.1082 (Calc. 458.1077 for C₁₆H₂₀N₅O₉P+H⁺).

**Compound 49. 5-[*N*-(2-[4-imidazole]ethyl)carboxyamide]cytidine monophosphate.** ¹H NMR (1:1 D₂O:CD₃OD) δ 3.06 (m, 2H), 3.60 (m, 1H), 3.68 (m, 1H), 4.12 (m, 2H), 4.25 (m, 1H), 4.29 (m, 2H), 5.95 (d, J = 3.8 Hz, 1H), 7.27 (s, 1H), 8.50 (s,1H), 8.53 (s, 1H). ¹³C NMR (1:1 D₂O:CD₃OD) δ 27.9, 67.5, 73.8, 79.1, 88.0, 93.7, 104.7, 120.2, 135.9, 136.8, 147.3, 159.3, 167.8, 170.1. ³¹P (1:5 CD₃OD:DMSO-d₆) δ 6.5. HRMS (FAB+) m/z 461.1186 (Calc. 461.1186 for C₁₅H₂₁N₆O₉P+H⁺).

**Compound 50. 5-[*N*-(2-[4-hydroxyphenyl]ethyl)carboxyamide]cytidine monophosphate.** ¹H NMR (D₂O) δ 2.82 (t, *J* = 6.8 Hz, 2H), 3.52 (m, 2H), 3.97 (m, 1H), 4.02 (m, 1H), 4.28 (m, 2H), 4.33 (t, *J = 4.6* Hz, 1H), 5.88 (d, *J* = 4.5 Hz, 1H), 6.83 (d, *J* = 8.4 Hz, 2H), 7.17 (d, *J* = 8.4 Hz, 2H), 8.24 (s, 1H). ¹³C NMR (D₂O) δ 34.8, 42.0, 64.4, 70.9, 75.7, 85.0, 91.1, 102.7, 116.2, 131.3, 132.3, 144.2, 155.1, 157.2, 164.7, 166.9. ³¹P NMR (D₂O) 7.8.

**Compound 51. 5-[*N*-(2-indolylethyl)carboxyamide]cytidine monophosphate.** ¹H NMR (CD₃OD) δ 2.94 (m, 2H), 3.55 (m, 2H), 4.04 (m, 1H), 4.11 (m, 2H), 4.20 (m, 2H), 5.92 (d, *J* = 4.2 Hz, 1H), 6.91 (m, 1H), 6.99 (m, 1H), 7.06 (s, 1H), 7.25 (d, *J* = 4.1 Hz, 1H), 7.61 (d, *J* = 7.5 Hz, 1H), 8.56 (s, 1H). MS (FAB-) m/z (M - H)- 507.7.

### Example 5

### 2'-Deoxycytidine Modifications with Amines

The following procedures were employed to produce the modified 2'-deoxycytidines shown in Table IV.

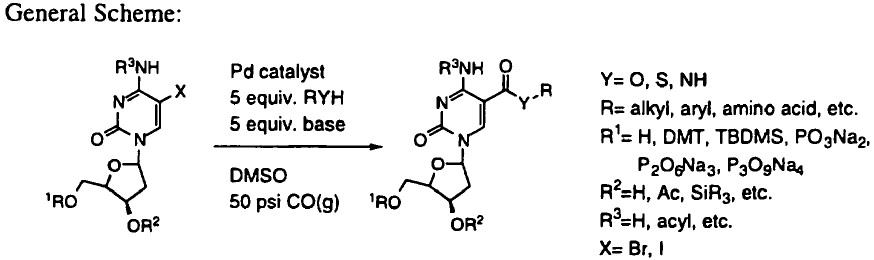

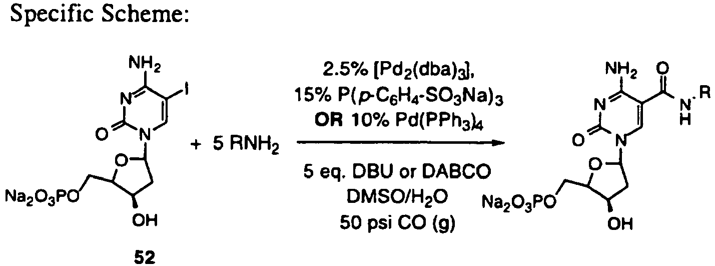

| RYH | |
|---|---|
| **A** | 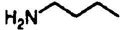 |
| **B** | 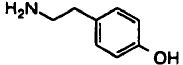 |

**Table IV. Summary of 2'-deoxycytidine carboxvamidation products.**

| Entry | Starting material | RNH₂ | Product ID | Isolated Yield (%) |
|---|---|---|---|---|
| 1^{b} | **52** | **A** | **53** | 26 |
| 2^{a} | **52** | **B** | **54** | 20 |

| | | | | |
|---|---|---|---|---|
| ^{a} Nucleotide (0.1 mmol), RNH₂ (0.5 mmol), Pd₂(dba)₃ (2.5 µmol), P(*p*-C₆H₄-SO₃Na)₃ (0.015 mmol), DABCO (0.7 mmol), DMSO (0.5 mL). ^{b} Nucleotide (0.1 mmol), RNH₂ (0.5 mmol), Pd(PPh₃)₄ (0.01 mmol), DBU or DABCO (0.5 mmol), DMSO:H₂O 93:7 (0.5 mL). | | | | |

**Compound 53. 5-(*N*-butylcarboxyamide)-2'-deoxycytidine monophosphate.** ¹H NMR (D₂O) δ 0.90 (t, *J* = 7.3 Hz, 3H), 1.35 (m, 2H), 1.58 (m, 2H), 2.30 (m, 1H), 2.52 (m, 1H), 3.31 (m, 2H), 4.11 (m, 2H), 4.26 (m, 1H), 4.54 (m, 1H), 6.22 (m, 1H), 8.41 (s, 1H).

**Compound 54. 5-[*N*-(2-[4-hydroxyphenyl]ethyl)carboxyamide]-2'-deoxycytidine monophosphate.** ¹H NMR (D₂O) δ 2.27 (m, 1H), 2.41 (m, 1H), 2.82 (m, 2H), 3.51 (m, 2H), 3.90 (m, 2H), 4.19 (m, 1H), 4.50 (m, 1H), 6.16 (m, 1H), 6.83 (d, *J* = 8.3 Hz, 2H), 7.16 (d, *J* = 8.3 Hz, 2H), 8.19 (s, 1H).

### Example 6

### Determination of Antiviral Efficacy and Cellular Toxicity

This example demonstrates the ability of nucleotides of the invention to inhibit cytopathologic effects associated with human cytomagalovirus (CMV) infection. The experimental drugs of this example will be referred to as CT1146-26 and CT1146-28. CT1146-26 is the monophosphate of Compound 30 and has the following structure:

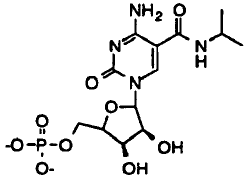

CT1146-28 is the monophosphate of Compound 41 and has the following structure:

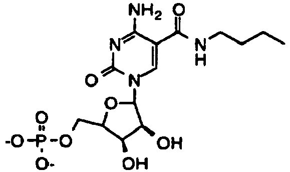

### Assay methodology and preliminary results are provided in the following sections. Cell Isolation and Tissue Culture

Newborn human foreskins were obtained immediately post-circumcision and placed in minimal essential medium (MEM) supplemented with vancomycin, fungizone, penicillin and gentamycin (all present at standard concentrations) and maintained for four hours under tissue culture conditions (37°C, 5% CO₂). Supplemented MEM was then removed and the foreskin material macerated. Tissue fragments were then washed exhaustively (using supplemented MEM) to remove residual erythrocyte contamination.

The erythrocyte-free cell fragments were then exposed to trypsin solution (0.25%) for 15 min with continuous stirring. Tissue fragments were then allowed to settle out of suspension and the supernatant collected and passed through sterile cheesecloth into a flask containing MEM and 10% fetal bovine serum (FBS). The cheesecloth was washed with an additional volume of MEM containing 10% FBS (The flask containing MEM and 10% FBS was maintained on ice throughout the trypsinization procedure). The trypsinization procedure was repeated using fresh trypsin solution, until no additional cell removal from tissue fragments was observed. Cells removed by this process are predominantly human foreskin fibroblasts (HFF).

Cells contained in MEM with 10% FBS were pelleted by centrifugation (≅ 1000 RPM, 4°C, 10 min), the supernatant discarded and the pelleted cells suspended in a minimal volume of MEM with 10% FBS. HFF isolates were then plated into T-25 tissue culture flasks (the number of primary culture flasks used was based on the volume of the recovered cell pellet). HFF isolates were then maintained under tissue culture conditions (37°C, 5% CO₂) until confluent. Primary cultures of HFF were sequentially expanded into larger format culture flasks using standard tissue culture procedures. HFF were maintained in the presence of vancomycin and fungizone through passage four.

### Cytotoxicity Assay: IC₅₀ Determination

HFF were seeded into 96-well tissue culture plates at a concentration of 2.5x10⁴ cells/ml (100µl MEM with 10% FBS was used as the culture medium) and maintained under tissue culture conditions for 24H prior to experimentation. Medium from plates was then removed and 100 µl of MEM containing 2% FBS was added to all but the first row of cells in the 96-well plate. To the first row of each 96-well plate, 125 µl of control media or experimental drug CT1146-26 or CT1146-28 (initial concentration determined by the overall concentration range desired) was added in triplicate wells. Medium alone was added to both cell and virus control wells. The contents of the first row of wells were then serially diluted (1:5) across the remaining rows of the plate (25 µl volume transfer well to well, with intermediate mixing, using a Cetus liquid handling machine). Following dilution, 100 µl of CMV (2500 PFU/well final concentration) in MEM with 2% FBS was added to each well of the 96-well plate, except for wells containing cell controls. Cell control wells received an additional 100 µl of MEM with 2% FBS. The 96-well plates were then incubated under tissue culture conditions (14 day total incubation period for CMV treated HFF in 2% FBS containing MEM; media addition to cultures was made as appropriate).

Following the incubation period, medium was removed from all wells and the cells stained with 0.1% crystal violet solution for 30 min followed by several wash cycles to remove residual stain. The crystal violet stained plates were then allowed to air dry for 24H prior to reading well absorbance values (620nm) using a Skatron plate reader. Cellular viability and corresponding IC₅₀ values were determined based on absorbance values for control and experimental drug treated cells relative to control cells which were not exposed to virus. IC₅₀ values (50% inhibitory concentration) for experimental drugs are determined as the concentration of drug required to inhibit cellular proliferation by 50%.

### Plaque Reduction Assay Using Semi-Solid Overlay: EC₅₀ Determination

HFF are plated into 6-well tissue culture plates and maintained under tissue culture conditions for approximately two days prior to use. On the date of assay, experimental drug solutions are prepared as 2X concentrations in 2X MEM. Serial dilutions of experimental drug are then performed (1:5) using 2X MEM. The approximate concentration range for experimental agents being 200 to 0.06 µg/ml. Each drug or control solution was then diluted 1:1 with 0.8% agarose solution. Following dilution with agarose solution, the final experimental drug concentration range was 100 to 0.03 µg/ml, with a final agarose overlay concentration of 0.4%. Viral material (CMV) was diluted in MEM with 10% FBS to yield a concentration of virus producing 20-30 plaques per well.

Media was removed from HFF cultures and 200 µl of virus containing media was added to each well (200 µl of MEM was added to control wells containing cells not exposed to virus) of each 6-well plate. The assay plates were then incubated for 1H with shaking every 15min. Aliquots (2 ml) of agarose/experimental drug mixture were then applied in duplicate to appropriate wells in 6-well culture plates. Control groups received 2ml aliquots of MEM/agarose in a 1:1 dilution. Plates containing HFF and the various treatment groups were then incubated under tissue culture conditions for 14 days. On days 4 and 8, an additional 1ml of 1:1 2X MEM:agarose mixture was added to each well.

Following incubations, HFF were stained for 4-6H with a 1.5% solution of neutral red. The neutral red/agarose/MEM mixture is aspirated and viral plaques counted using a 10X steriomicroscope. EC₅₀ values (50% effective concentration) for each experimental drug are then determined as the concentration of experimental drug required to inhibit viral cytopathogenicity by 50%.

The Selectivity Index (SI) for each drug treatment was also determined (SI = IC₅₀/ EC₅₀). Increasing efficacy of experimental drug in the absence of equivalent cytotoxicity of the drug, will result in increasing SI ratios (ie. A candidate compound with an IC₅₀ of 1 µg/ml and an EC₅₀ of 0.01 µg/ml will have an SI = 100; Conversely a candidate compound with an IC₅₀ of 0.01 µg/ml and an EC₅₀ of 1 µg/ml will have an SI = 0.01).

### Results

Initial studies using nucleosides CT1146-28 and CT1146-26 suggest substantive anti-viral activity with respect to CMV as shown in Table V:

**Table V.**

| Compound | EC₅₀ (µg/ml) | IC₅₀ (µg/ml) | SI |
|---|---|---|---|
| CT1146-28 | < 0.03 | > 100 | > 3333 |
| CT1146-26 | < 0.03 | > 100 | > 3333 |

| | | | |
|---|---|---|---|
| EC₅₀ and IC₅₀ values are against human cytomegalovirus (CMV) | | | |

The novel nucleosides CT1146-28 and CT1146-26 exhibit significant anti-viral activity with respect to human CMV. Given the substantive differences between effective concentrations and cytotoxicity for these agents, it is unlikely that observed results reflect de facto cellular toxicity in response to treatment with these agents.

## Claims

1. A method for the preparation of a modified nucleoside comprising the steps of:
reacting a nucleoside starting material containing a leaving group attached to a carbon atom of said nucleoside starting material with a nucleophile and carbon monoxide in the presence of a palladium catalyst; and isolating said modified nucleoside, wherein said nucleophile is an amine, alcohol or thiol.

2. The method of Claim 1, wherein said nucleoside starting material is an adenine or guanine containing a leaving group attached to the 2-, 6- or 8-position

3. The method of Claim 1 or 2, wherein said adenine or guanine is 8-halo-adenine or 8-halo-guanine.

4. The method of Claim 3, wherein said halo group is iodo or bromo.

5. The method of Claim 1, wherein said nucleoside starting material is a cytidine or uridine containing a leaving group attached to the 5- or 6-position.

6. The method of Claim 5, wherein said cytidine or uridine is 5-halo-cytidine or 5-halo-uridine.

7. The method of Claim 5, wherein said halo group is iodo or bromo and the nucleoside is unsubstituted at the 5'-position or in the form of the 5'-monophosphate.

8. The method of Claim 6, wherein the said 5-halo-uridine is 5'-DMT-5-iodo-2',3'-O-isopropylideneuridine or 5'-TBDMS-5-iodo-2',3'-O-isopropylideneuridine.

9. The method of Claim 1, wherein said nucleophile has the formula RYH, wherein,
Y is selected from the group consisting of O, S, NH and NR'; and
each of R and R' is independently a substituted or unsubstituted C1-C20 alkyl (straight chain or branched), C2-C20 alkenyl (straight chain or branched), aryl or an amino acid residue, wherein R and R' can optionally be part of a cyclic structure which can be aromatic, aliphatic or heterocyclic.

10. The method of Claim 9, wherein each of R and R' is independently substituted by an amide, ester, nitrile, nitro, urea halide, cyanate, alcohol, amine, ether, thiol or aryl group.

11. The method of Claim 9, wherein
Y is selected from the group consisting of O, S, and NH;
R is (CH₁)ₘ(CH₃)ₙ, wherein I is 0, 1, or 2; m is 0-19; n is 0, 1, 2, or 3; and wherein one or more of the H are optionally substituted with =O, -OH, =NH, NH₂, ⁺NMe₃Cl, 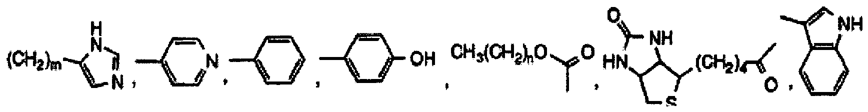 or an amino acid.

12. The method of Claim 9, wherein said nucleophile is selected from the group consisting of:

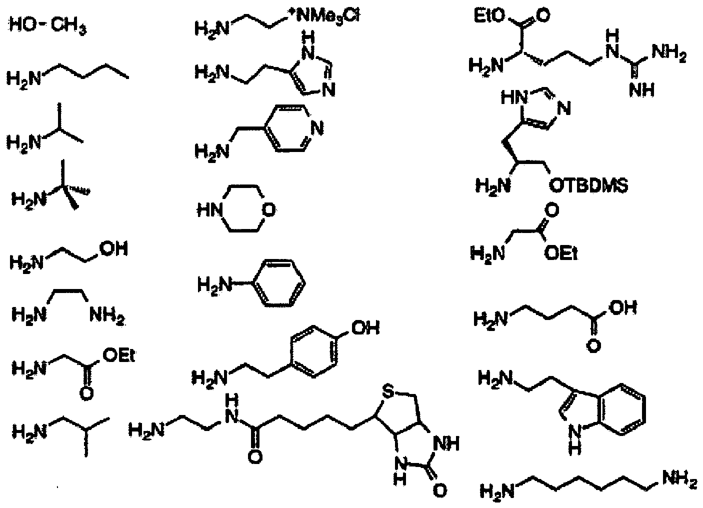

13. The method of any preceding Claim, wherein said palladium catalyst is of the formula PdL₃ or PdL₄, wherein L is selected from the group consisting of P(C₆H₅)₃, P(p-C₆H₄SO₃Na)₃, (o-tol)₃P, CH₃CN, DMSO, N,N-dimethylformamide (DMF),

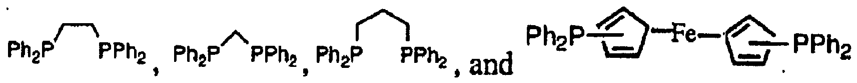

14. The method of Claim 13, wherein said palladium catalyst is Pd(P(C₆H₅)₃)₄ or P(p-C₆H₄SO₃Na)₃.

15. The method of any preceding Claim, wherein said leaving group is selected from the group consisting of a halogen, acetate, trifluoroacetate, tosylate, methylsulfonate, trifluoromethyl sulfonate, boronic ester and boronic acid.

16. A compound selected from
(A) 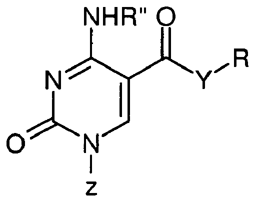 wherein
Y is selected from the group consisting of S and NH; and
R is selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R can optionally be part of a cyclic structure which can be aromatic, aliphatic, or heterocyclic;
R" is selected from the group consisting of H and acyl; and
Z is selected from the group consisting of a ribose, deoxyribose, dideoxyribose, and any combination of 2', 3', and 5' modifications thereof;
(B) 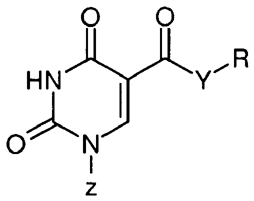 wherein
Y is selected from the group consisting of S, NH and NR'; and
R and R' are independently selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R and R' can optionally be part of a cyclic structure which can be aromatic, aliphatic, or heterocyclic;
R" is selected from the group consisting of H and acyl; and
Z is selected from the group consisting of a ribose, deoxyribose, dideoxyribose, and any combination of 2', 3', and 5' modifications thereof;
and
(C) the group consisting of: 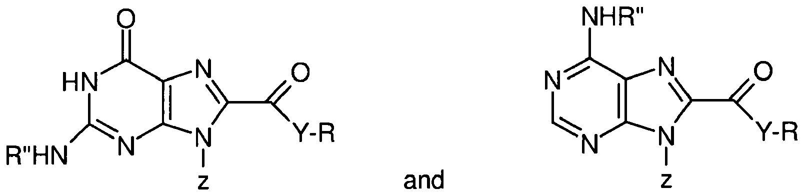 wherein
Y is selected from the group consisting of S and NH; and
R is independently selected from the group consisting of substituted or unsubstituted C1-C20 alkyl (straight chain or branched), C2-C20 alkenyl (straight chain or branched), aryl, heterocyclic, natural amino acid and unnatural amino acid residues, wherein R can optionally be part of a cyclic structure which can be aromatic, aliphatic, or heterocyclic;
R" is selected from the group consisting of H and acyl; and
Z is selected from the group consisting of a ribose, deoxyribose, dideoxyribose, and any combination of 2', 3', and 5' modifications thereof.

17. A compound of Claim 16, wherein
R is C1-C20 alkyl and is (CH₁)ₘ(CH₃)ₙ, wherein I is 0, 1,or 2; m is 0- 19; n is 1, 2,or 3; and wherein one or more of the H are optionally substituted with =O, -OH, =NH, NH₂, ⁺NMe₃Cl, 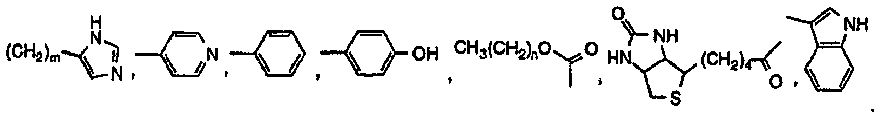 or an amino acid,
and
for compounds (B):
Y is selected from the group consisting of S and NH;

18. A compound of Claim 17 selected from (A), (B) and (C), wherein Y is NH and R is a C1-C20 alkyl.

19. A compound of Claim 18 selected from the group consisting of

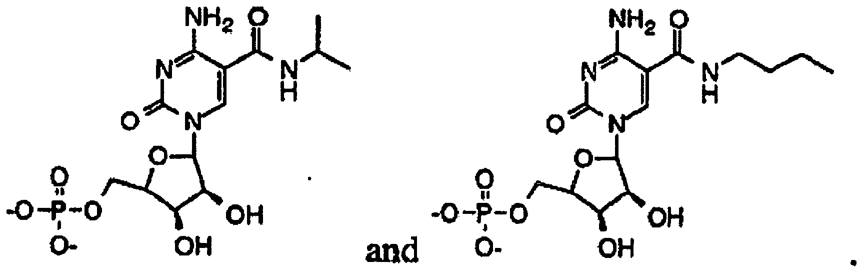

20. A compound of Claim 16 selected from the group consisting of

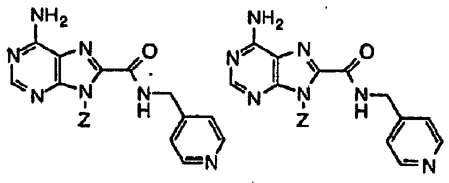

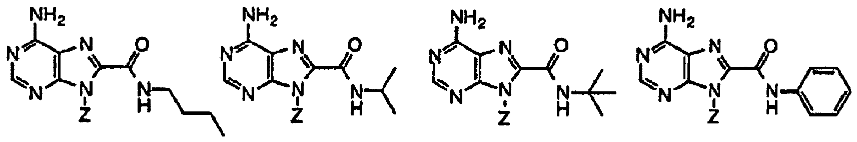

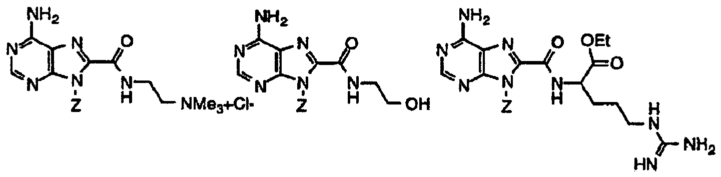

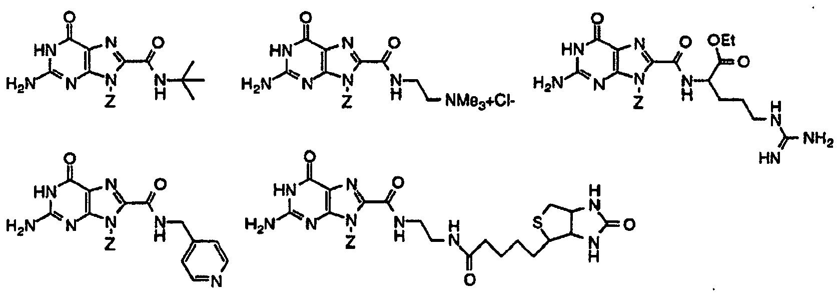

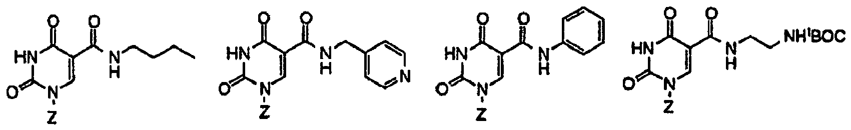

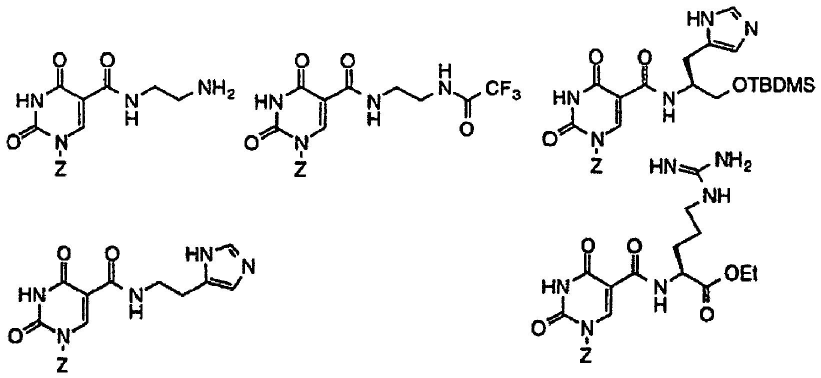

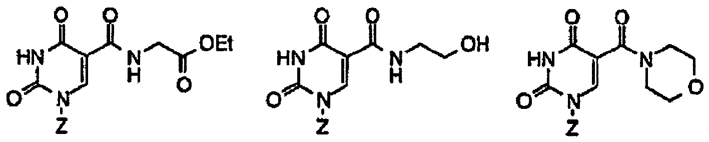

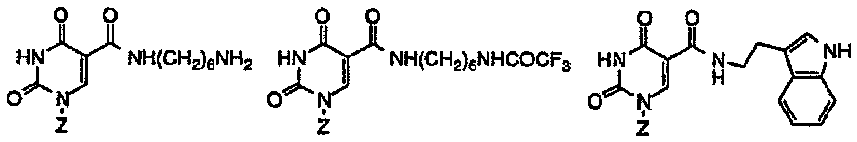

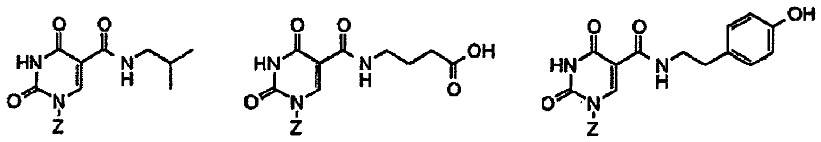

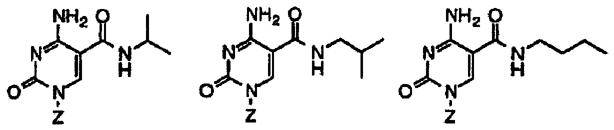

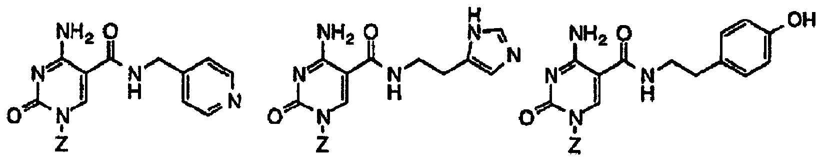

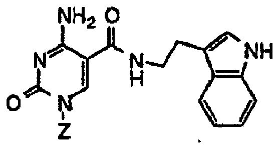

21. A compound of Claim 16 selected from the group consisting of

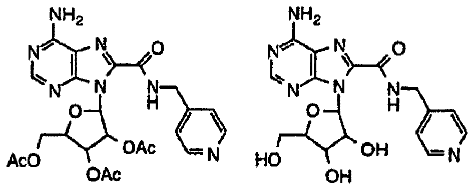

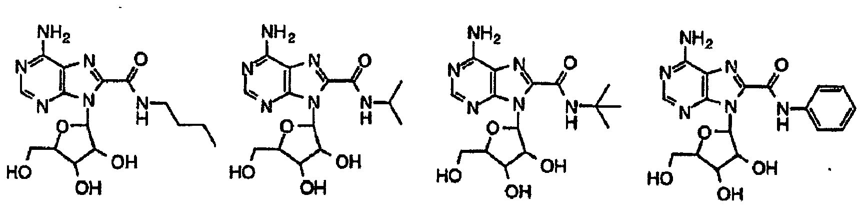

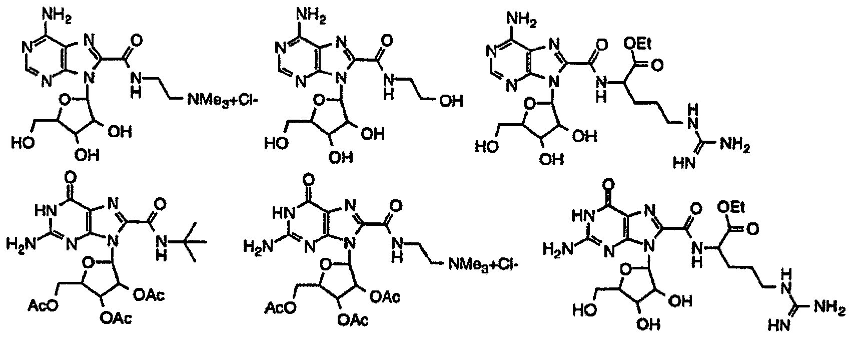

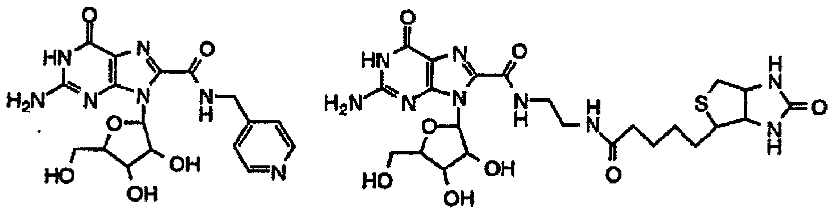

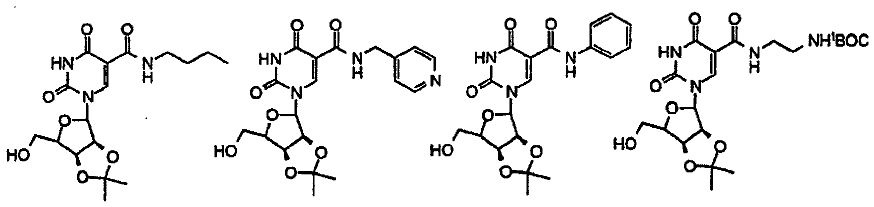

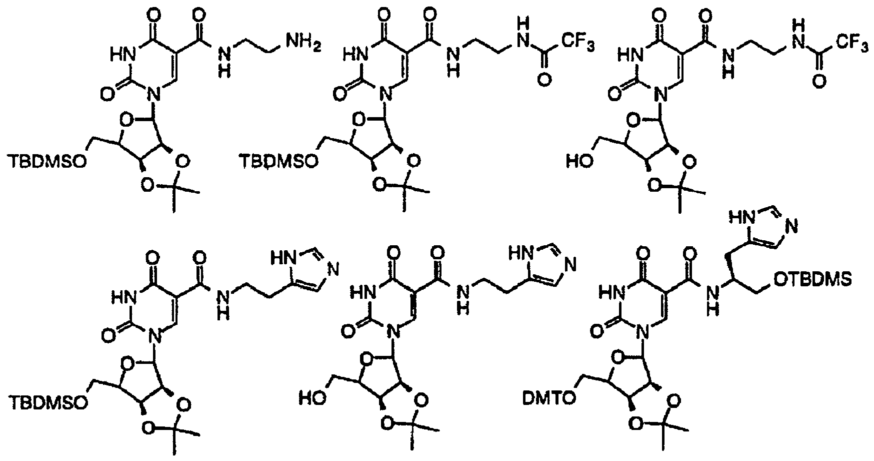

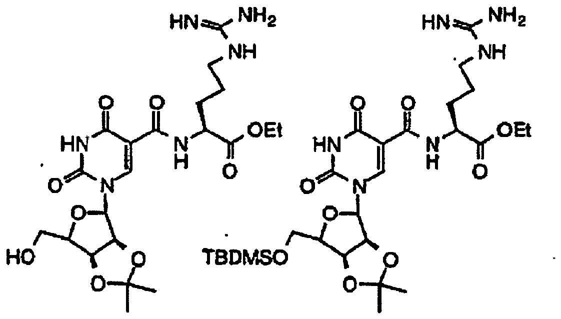

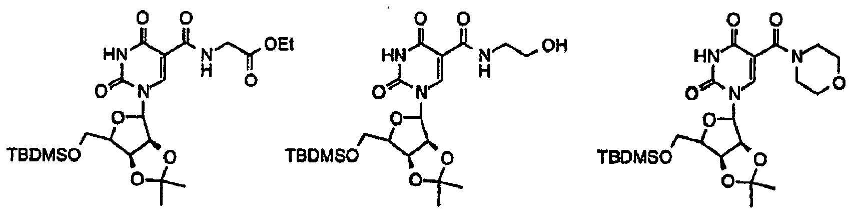

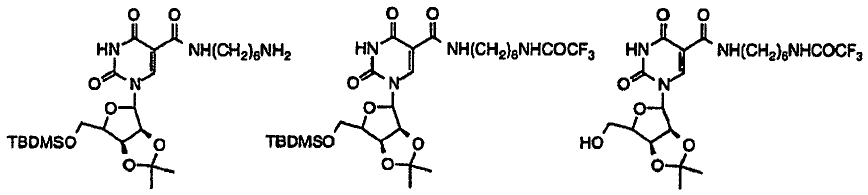

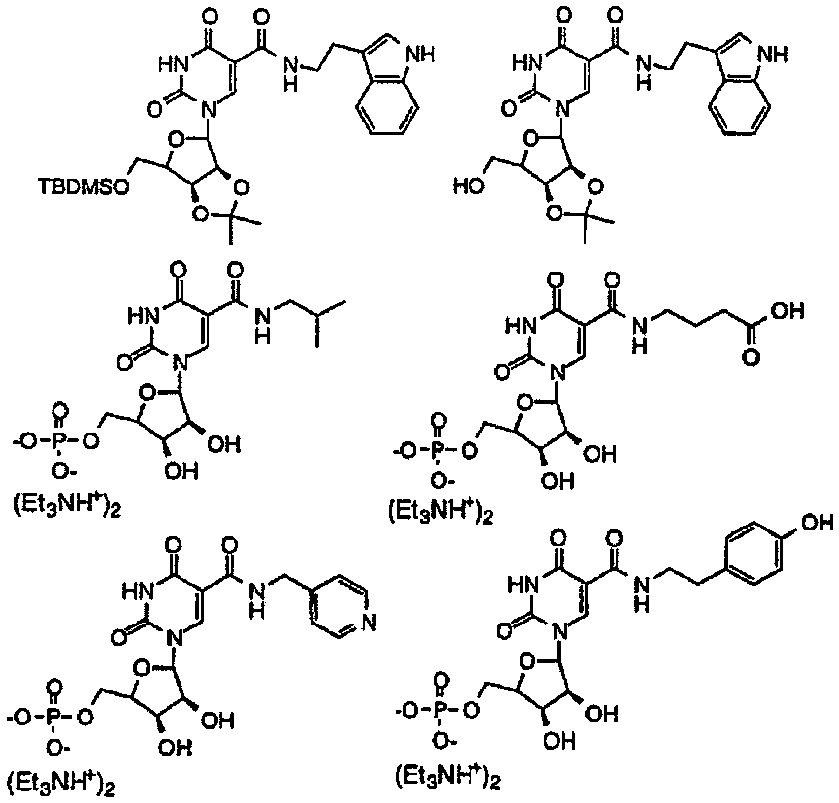

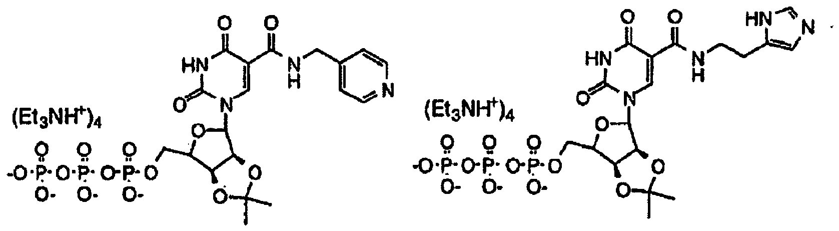

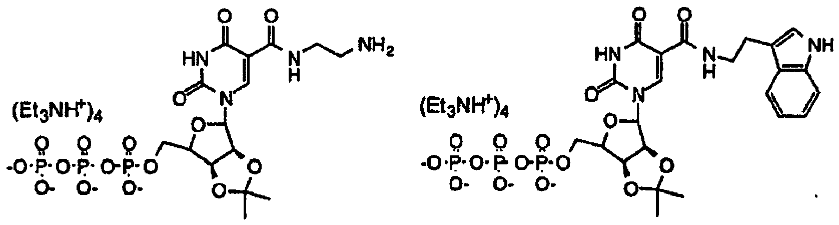

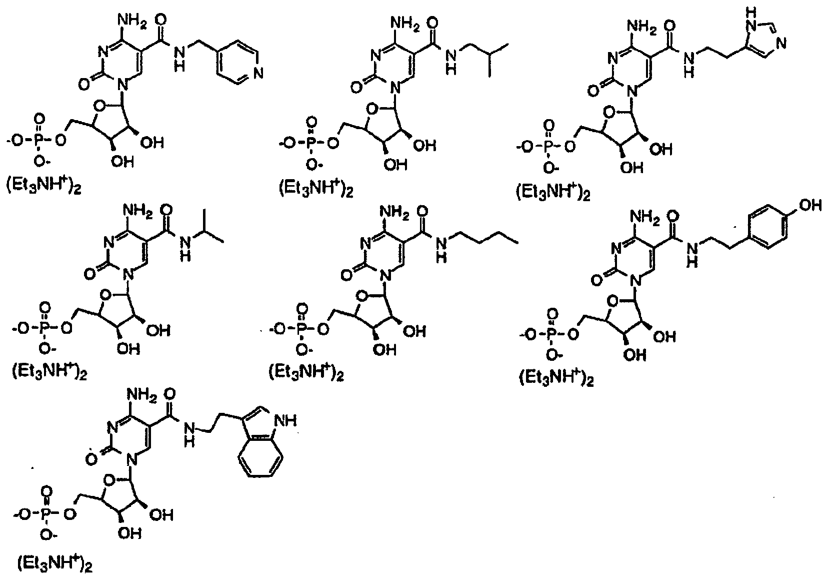

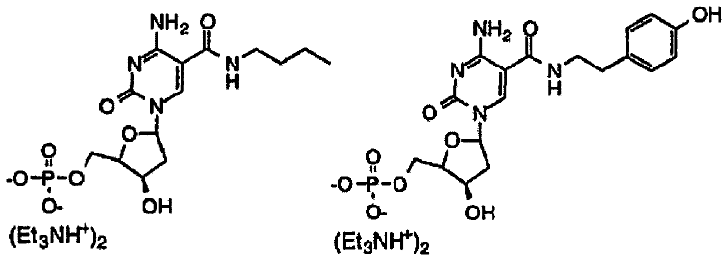

22. An oligoribonucleotide or oligodeoxyribonucleotide containing one or more nucleotides derived from a modified nucleoside claimed in Claim 16, 17, 18, 19, 20, or 21.

23. A compound claimed in Claim 16, 17, 18, 19, 20, or 21 for use in therapy.

24. A compound claimed in Claim 16, 17, 18, 19, 20, or 21, for inhibiting or treating cytomegalovirus activity.

25. Use of a compound claimed in Claim 16, 17, 18, 19, 20, or 21, in the preparation of a medicament for use in inhibiting or treating cytomegalovirus activity.

## Patentansprüche

1. Verfahren zur Herstellung eines modifizierten Nucleosids, das die folgenden Schritte umfasst:
Umsetzen eines Nucleosid-Ausgangsmaterials, das eine an ein Kohlenstoffatom des Nucleosid-Ausgangsmaterials gebundene Abgangsgruppe umfasst, mit einem Nucleophil und Kohlenstoffmonoxid in Gegenwart eines Palladiumkatalysators; und Isolieren des modifizierten Nucleosids, wobei das Nucleophil ein Amin, Alkohol oder Thiol ist.

2. Verfahren nach Anspruch 1, wobei das Nucleosid-Ausgangsmaterial ein Adenin oder Guanin mit einer an Position 2, 6 oder 8 gebundenen Abgangsgruppe ist.

3. Verfahren nach Anspruch 1 oder 2, wobei das Adenin oder Guanin 8-Halogenadenin oder 8-Halogenguanin ist.

4. Verfahren nach Anspruch 3, wobei die Halogengruppe Iod oder Brom ist.

5. Verfahren nach Anspruch 1, wobei das Nucleosid-Ausgangsmaterial ein Cytidin oder Uridin mit einer an Position 5 oder 6 gebundenen Abgangsgruppe ist.

6. Verfahren nach Anspruch 5, wobei das Cytidin oder Uridin 5-Halogencytidin oder 5-Halogenuridin ist.

7. Verfahren nach Anspruch 5, wobei die Halogengruppe Iod oder Brom ist und das Nucleosid an Position 5' unsubstituiert ist oder in Form des 5'-Monophosphats vorliegt.

8. Verfahren nach Anspruch 6, wobei das 5-Halogenuridin 5'-DMT-5-iod-2',3'-O-isopropylidenuridin oder 5'-TBDMS-5-iod-2',3'-O-isopropylidenuridin ist.

9. Verfahren nach Anspruch 1, wobei das Nucleophil die Formel RYH aufweist, wobei
Y aus der aus O, S, NH und NR' bestehenden Gruppe ausgewählt ist; und
R und R' unabhängig voneinander substituiertes oder unsubstituiertes C₁-C₂₀-Alkyl (unverzweigt oder verzweigt), C₂-C₂₀-Alkenyl (unverzweigt oder verzweigt), Aryl oder ein Aminosäurerest sind, wobei R und R' gegebenenfalls Teil einer zyklischen Struktur sind, die aromatisch, aliphatisch oder heterozyklisch sein kann.

10. Verfahren nach Anspruch 9, wobei R und R' jeweils unabhängig voneinander mit einer Amid-, Ester-, Nitril-, Nitro-, Harnstoffhalogenid-, Cyanat-, Alkohol-, Amino-, Ether-, Thiol- oder Arylgruppe substituiert sind.

11. Verfahren nach Anspruch 9, wobei
Y aus der aus O, S und NH bestehenden Gruppe ausgewählt ist;
R (CH₁)ₘ(CH₃)ₙ ist, worin I = 0, 1 oder 2 ist; m = 0-19 ist; n = 0, 1, 2 oder 3 ist; und wobei eines oder mehrere der H gegebenenfalls durch =O, -OH, =NH, NH₂, ⁺NMe₃Cl, 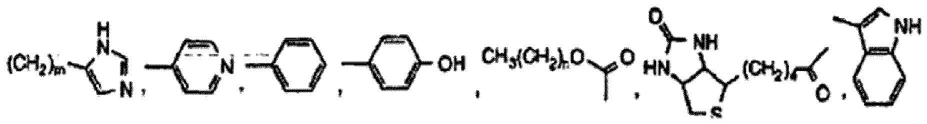 oder eine Aminosäure ersetzt sind.

12. Verfahren nach Anspruch 9, wobei das Nucleophil aus folgender Gruppe ausgewählt ist:

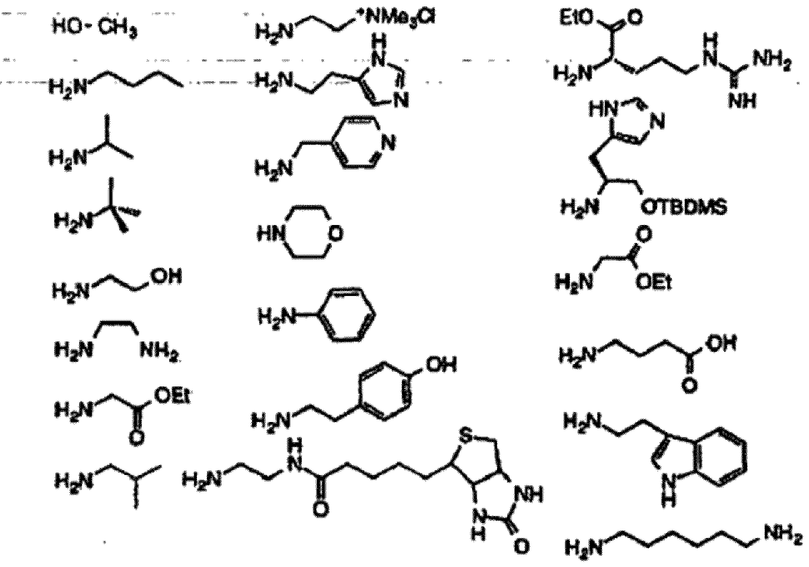

13. Verfahren nach einem der vorangegangenen Ansprüche, wobei der Palladiumkatalysator die Formel PdL₃ oder PdL₄ aufweist, worin L aus der aus P(C₆H₅)₃, P(p-C₆H₄SO₃Na)₃, (o-tol)₃P, CH₃CN, DMSO, N,N-Dimethylformamid (DMF), 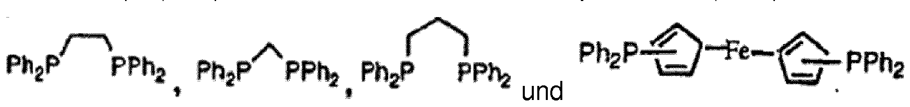 bestehenden Gruppe ausgewählt ist.

14. Verfahren nach Anspruch 13, wobei der Palladiumkatalysator Pd(P(C₆H₅)₃)₄ oder p(p-C₆H₄SO₃Na)₃ ist.

15. Verfahren nach einem der vorangegangenen Ansprüche, wobei die Abgangsgruppe aus der aus Halogen, Acetat, Trifluoracetat, Tosylat, Methylsulfonat, Trifluormethylsulfonat, Boronsäureester und Boronsäure bestehenden Gruppe ausgewählt ist.

16. Verbindung, ausgewählt aus
(A) 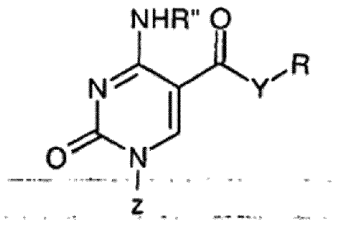 worin
Y aus der aus S und NH bestehenden Gruppe ausgewählt ist;
R aus der aus substituiertem oder unsubstituiertem C₁-C₂₀-Alkyl (unverzweigt oder verzweigt), C₂-C₂₀-Alkenyl (unverzweigt oder verzweigt), Aryl, Heterocyclyl, natürlichen Aminosäure- und nichtnatürlichen Aminosäureresten bestehenden Gruppe ausgewählt ist, wobei R gegebenenfalls Teil einer zyklischen Struktur ist, die aromatisch, aliphatisch oder heterozyklisch sein kann;
R" aus der aus H und Acyl bestehenden Gruppe ausgewählt ist; und
Z aus der aus einer Ribose, Desoxyribose, Didesoxyribose und einer beliebigen Kombination aus 2'-, 3'- und 5'-Modifikationen davon bestehenden Gruppe ausgewählt ist;
(B) 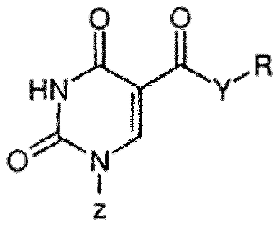 worin
Y aus der aus S, NH und NR' bestehenden Gruppe ausgewählt ist;
R und R' aus der aus substituiertem oder unsubstituiertem C₁-C₂₀-Alkyl (unverzweigt oder verzweigt), C₂-C₂₀-Alkenyl (unverzweigt oder verzweigt), Aryl, Heterocyclyl, natürlichen Aminosäure- und nichtnatürlichen Aminosäureresten bestehenden Gruppe ausgewählt ist, wobei R gegebenenfalls Teil einer zyklischen Struktur ist, die aromatisch, aliphatisch oder heterozyklisch sein kann;
R" aus der aus H und Acyl bestehenden Gruppe ausgewählt ist; und
Z aus der aus einer Ribose, Desoxyribose, Didesoxyribose und einer beliebigen Kombination aus 2'-, 3'- und 5'-Modifikationen davon bestehenden Gruppe ausgewählt ist;
und
(C) folgender Gruppe: 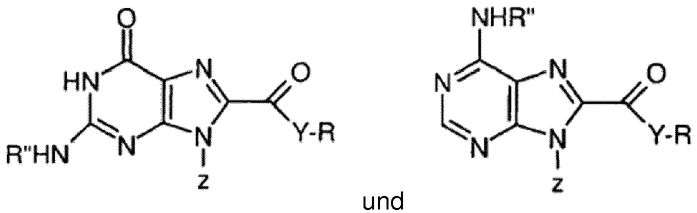 worin
Y aus der aus S und NH bestehenden Gruppe ausgewählt ist;
R unabhängig aus der aus substituiertem oder unsubstituiertem C₁-C₂₀-Alkyl (unverzweigt oder verzweigt), C₂-C₂₀-Alkenyl (unverzweigt oder verzweigt), Aryl, Heterocyclyl, natürlichen Aminosäure- und nichtnatürlichen Aminosäureresten bestehenden Gruppe ausgewählt ist, wobei R gegebenenfalls Teil einer zyklischen Struktur ist, die aromatisch, aliphatisch oder heterozyklisch sein kann;
R" aus der aus H und Acyl bestehenden Gruppe ausgewählt ist; und
Z aus der aus einer Ribose, Desoxyribose, Didesoxyribose und einer beliebigen Kombination aus 2'-, 3'- und 5'-Modifikationen davon bestehenden Gruppe ausgewählt ist.

17. Verbindung nach Anspruch 16, worin
R C₁-C₂₀-Alkyl ist und (CH₁)ₘ(CH₃)ₙ ist, worin I = 0, 1 oder 2 ist; m = 0-19 ist; n = 0, 1, 2 oder 3 ist; und wobei eines oder mehrere der H gegebenenfalls durch =O, -OH, =NH, NH₂, ⁺NMe₃Cl, 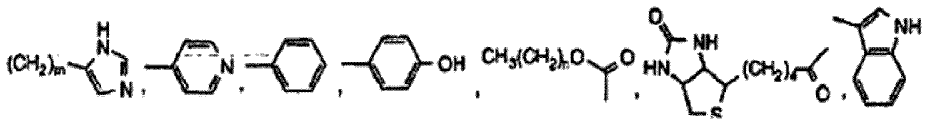 oder eine Aminosäure ersetzt sind
und
Y in Verbindungen (B) aus der aus S und NH bestehenden Gruppe ausgewählt ist.

18. Verbindung nach Anspruch 17, die aus (A), (B) und (C) ausgewählt ist, worin Y NH ist und R ein C₁-C₂₀-Alkyl ist.

19. Verbindung nach Anspruch 18, die aus folgender Gruppe ausgewählt ist:

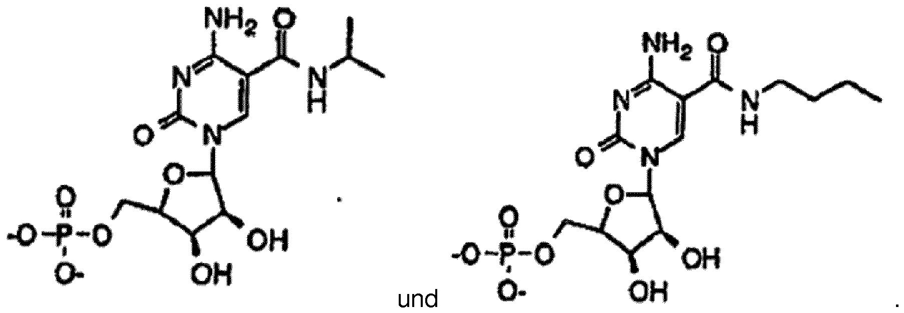

20. Verbindung nach Anspruch 16, die aus folgender Gruppe ausgewählt ist:

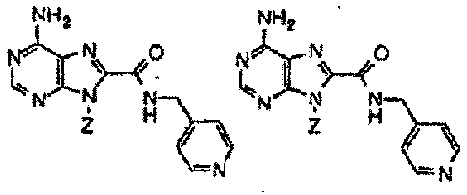

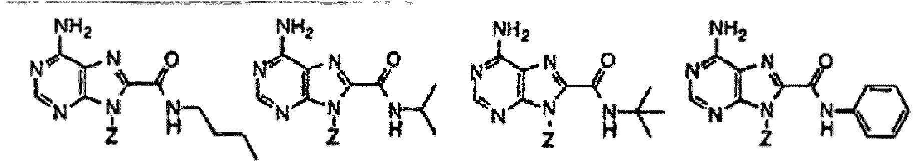

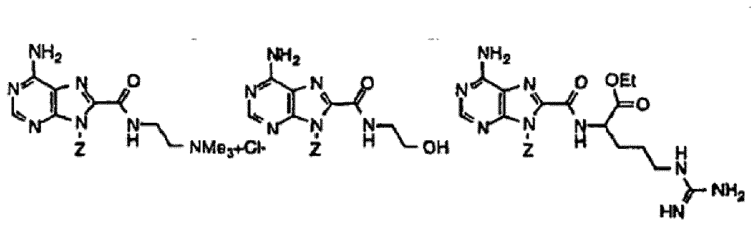

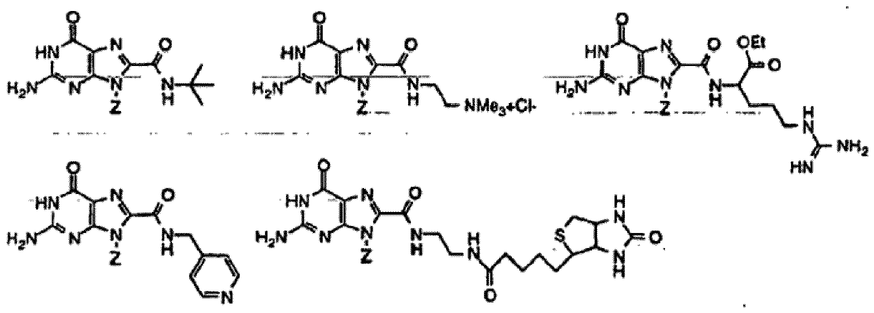

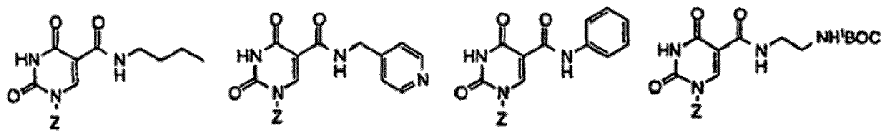

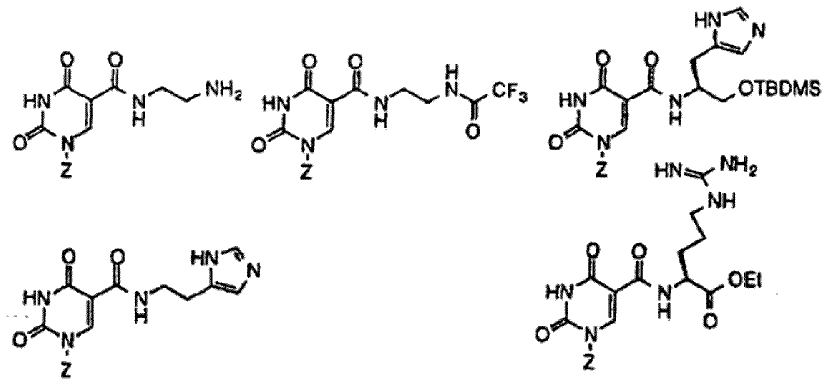

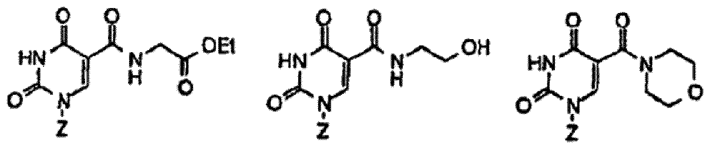

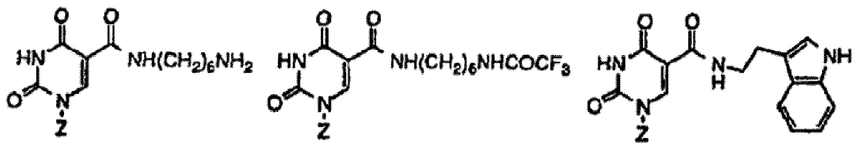

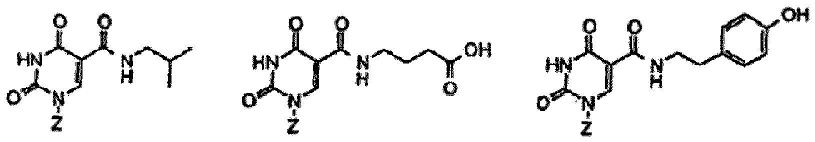

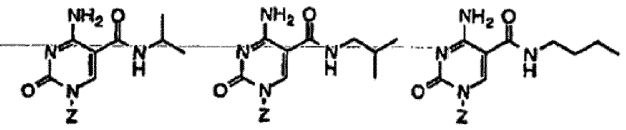

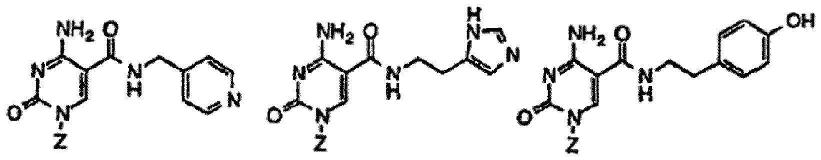

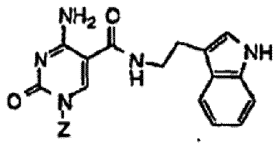

21. Verbindung nach Anspruch 16, die aus folgender Gruppe ausgewählt ist:

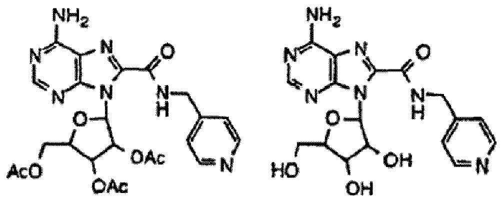

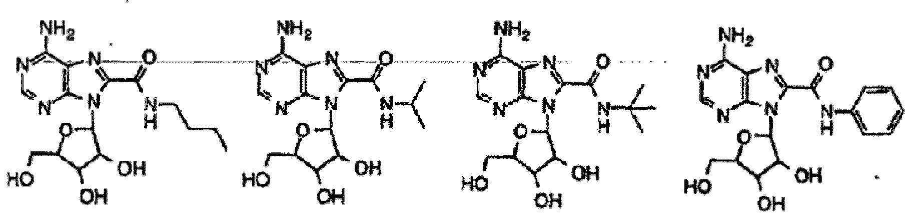

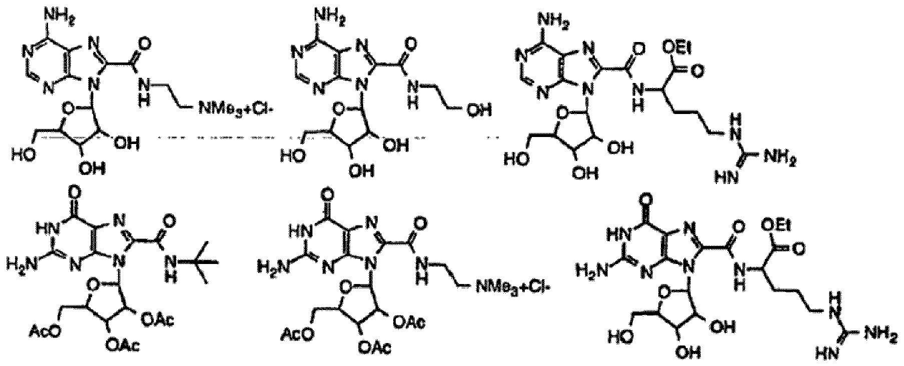

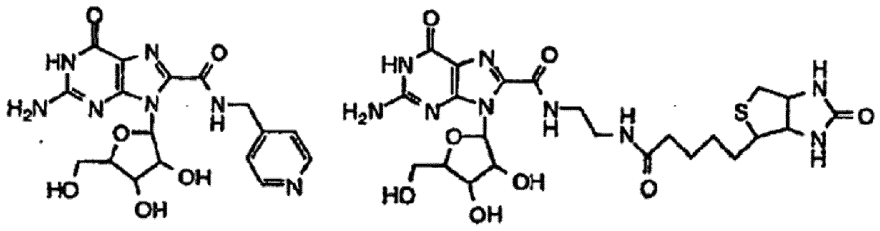

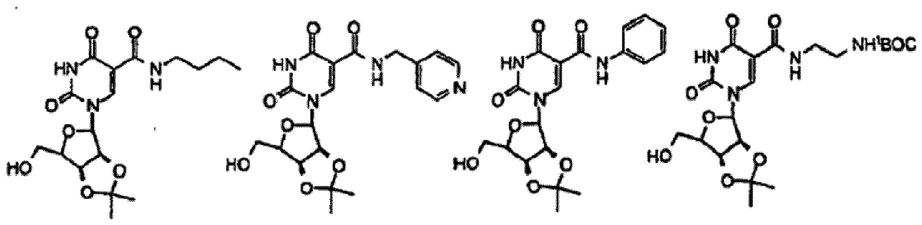

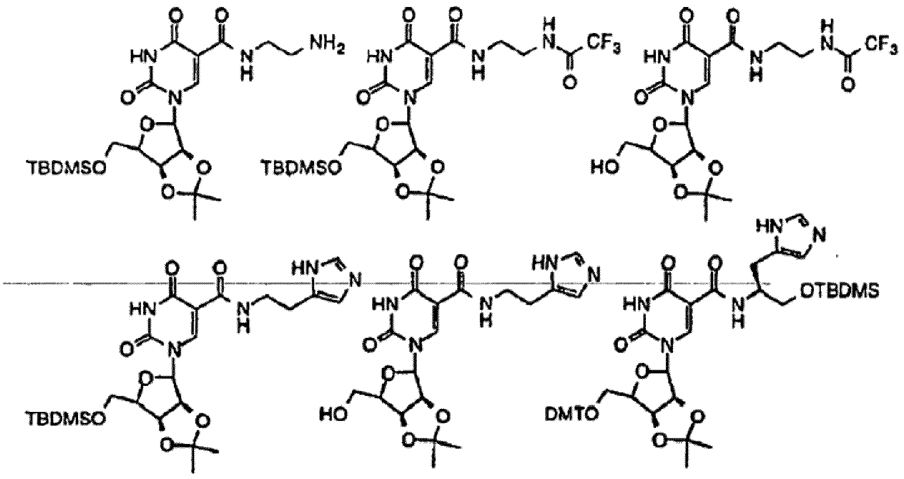

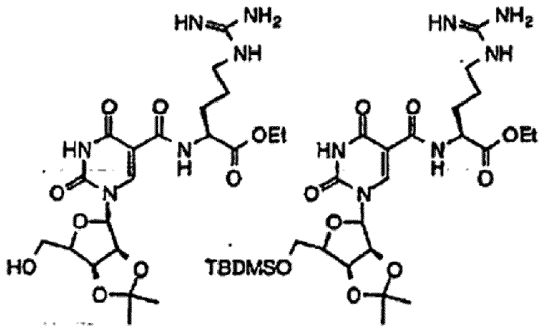

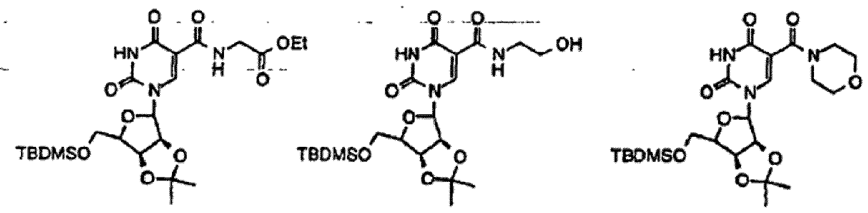

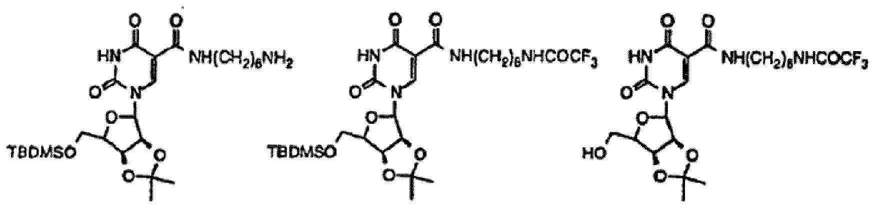

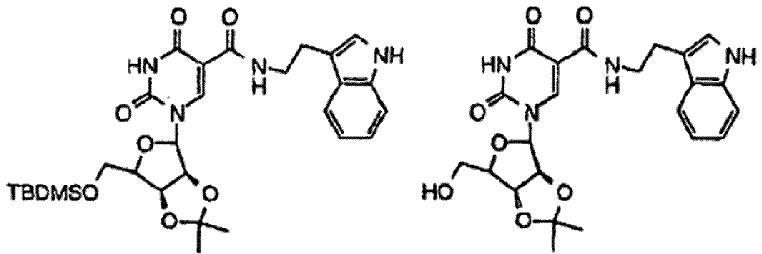

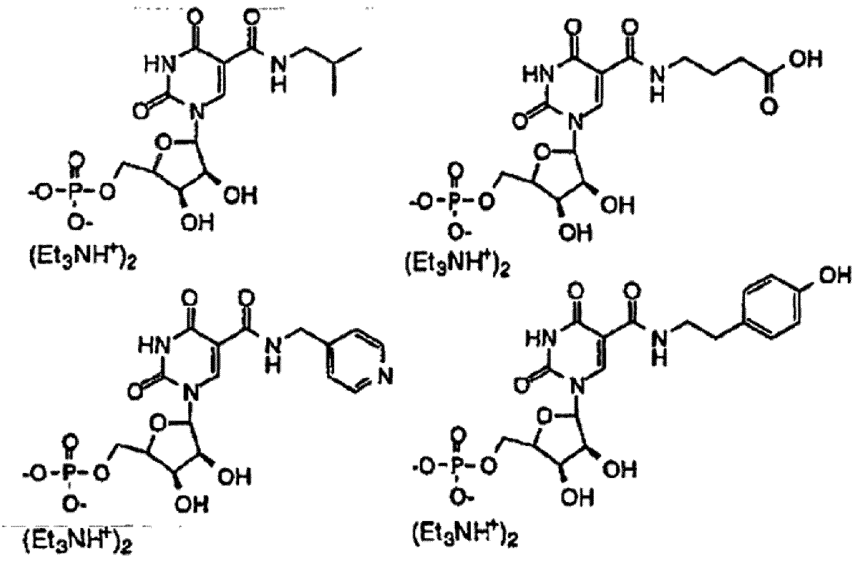

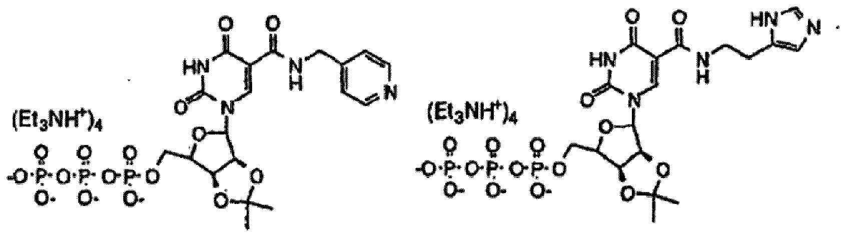

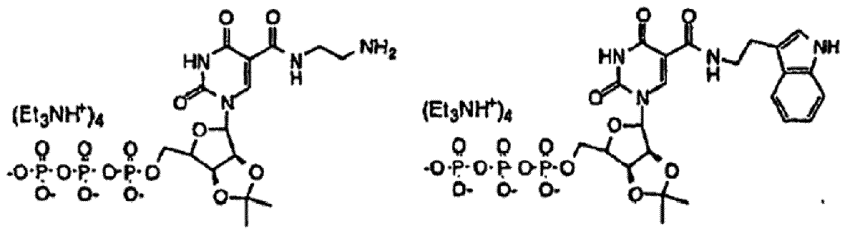

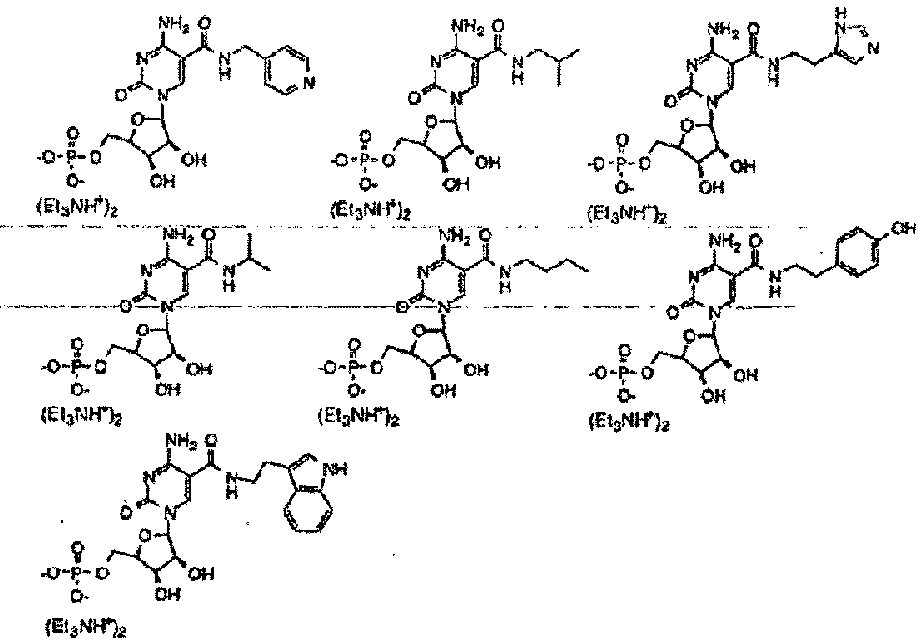

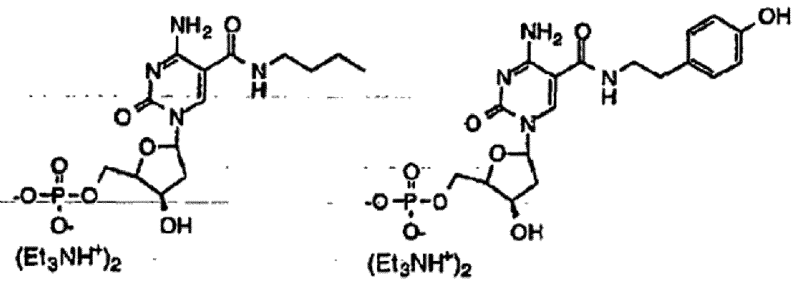

22. Oligoribonucleotid oder Oligodesoxyribonucleotid, das ein oder mehrere Nucleotide umfasst, die von einem in einem der Ansprüche 16, 17, 18, 19, 20 oder 21 beanspruchten modifizierten Nucleosid abgeleitet sind.

23. Verbindung nach Anspruch 16, 17, 18, 19, 20 oder 21 zur Verwendung in einer Therapie.

24. Verbindung nach Anspruch 16, 17, 18, 19, 20 oder 21 zur Hemmung oder Behandlung von Zytomegalievirus-Aktivität.

25. Verwendung einer Verbindung nach einem der Ansprüche 16, 17, 18, 19, 20 oder 21 bei der Herstellung eines Medikaments zur Hemmung oder Behandlung von Zytomegalievirus-Aktivität.

## Revendications

1. Procédé pour la préparation d'un nucléoside modifié, comprenant les étapes consistant à :
faire réagir un matériau de départ nucléoside contenant un groupe partant rattaché à un atome de carbone dudit matériau de départ nucléoside avec un nucléophile et du monoxyde de carbone en présence d'un catalyseur au palladium ; et isoler ledit nucléoside modifié, dans lequel ledit nucléophile est une amine, un alcool ou un thiol.

2. Procédé selon la revendication 1, dans lequel ledit matériau de départ nucléoside est une adénine ou guanine contenant un groupe partant rattaché à la position 2, 6 ou 8.

3. Procédé selon la revendication 1 ou 2, dans lequel ladite adénine ou guanine est une 8-halogénoadénine ou 8-halogénoguanine.

4. Procédé selon la revendication 3, dans lequel ledit groupe halogéno est iodo ou bromo.

5. Procédé selon la revendication 1, dans lequel ledit matériau de départ nucléoside est une cytidine ou uridine contenant un groupe partant rattaché à la position 5 ou 6.

6. Procédé selon la revendication 5, dans lequel ladite cytidine ou uridine est une 5-halogénocytidine ou 5-halogénouridine.

7. Procédé selon la revendication 5, dans lequel ledit groupe halogéno est iodo ou bromo et le nucléoside est non substitué en position 5' ou sous la forme du 5'-monophosphate.

8. Procédé selon la revendication 6, dans lequel ladite 5-halogénouridine est la 5'-DMT-5-iodo-2',3'-O-isopropylidène-uridine ou la 5'-TBDMS-5-iodo-2',3'-O-isopropylidène-uridine.

9. Procédé selon la revendication 1, dans lequel ledit nucléophile est de formule RYH dans laquelle Y est choisi dans le groupe constitué par 0, S, NH et NR' ; et
chaque R ou R' est indépendamment un radical substitué ou non substitué alkyle en C₁ à C₂₀ (linéaire ou ramifié), alcényle en C₂ à C₂₀ (linéaire ou ramifié), aryle, ou un résidu d'acide aminé, R et R' pouvant éventuellement faire partie d'une structure cyclique qui peut être aromatique, aliphatique ou hétérocyclique.

10. Procédé selon la revendication 9, dans lequel chacun de R et R' est indépendamment substitué par un groupe amide, ester, nitrile, nitro, urée halogénure, cyanate, alcool, amine, éther, thiol ou aryle.

11. Procédé selon la revendication 9, dans lequel Y est choisi dans le groupe constitué par 0, S et NH ;
R est (CH₁)ₘ(CH₃)ₙ où 1 vaut 0, 1 ou 2 ; m vaut 0-19 ; n vaut 0, 1, 2 ou 3 ; et
dans lequel un ou plusieurs des H sont éventuellement remplacés par =O, -OH, =NH, NH₂, ⁺NMe₃Cl, 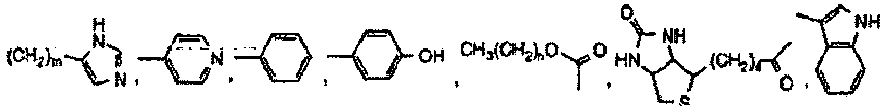 ou un acide aminé.

12. Procédé selon la revendication 9, dans lequel ledit nucléophile est choisi dans le groupe constitué par :

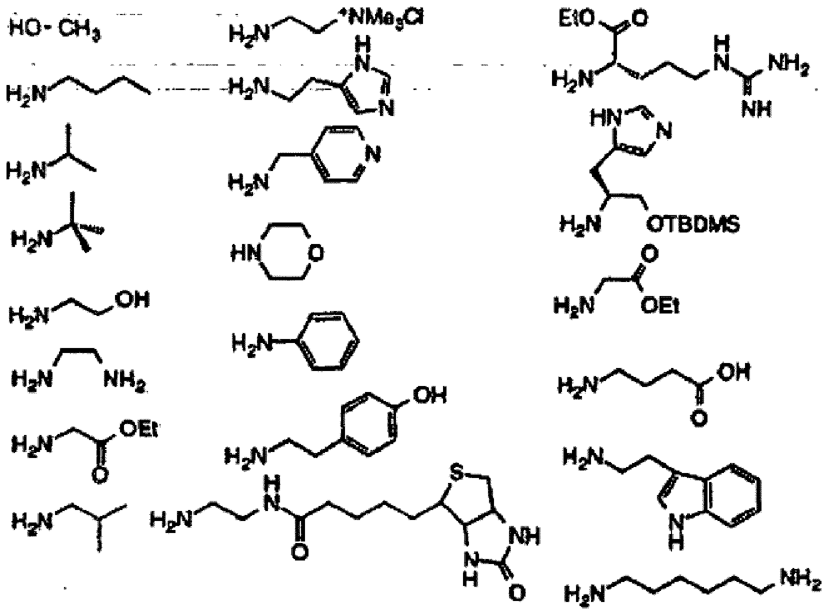

13. Procédé selon l'une quelconque des revendications précédentes, dans lequel ledit catalyseur au palladium est de formule PdL₃ ou PdL₄ où L est choisi dans le groupe constitué par P(C₆H₅)₃, P(p-C₆H₄SO₃Na)₃, (o-tol)₃P, CH₃CN, le DMSO, le N,N-diméthylformamide (DMF), 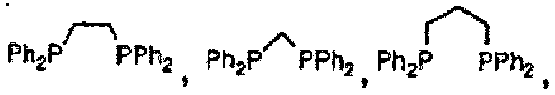 et

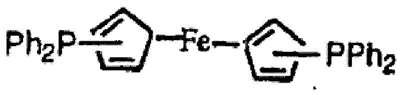

14. Procédé selon la revendication 13, dans lequel ledit catalyseur au palladium est Pd(P(C₆H₅)₃)₄ ou P(p-C₆H₄SO₃Na)₃.

15. Procédé selon l'une quelconque des revendications précédentes, dans lequel ledit groupe partant est choisi dans le groupe constitué par un halogène, un acétate, un trifluoroacétate, un tosylate, un méthylsulfonate, un trifluorométhylsulfonate, un ester boronate et l'acide boronique.

16. Composé choisi parmi les suivants :
(A) 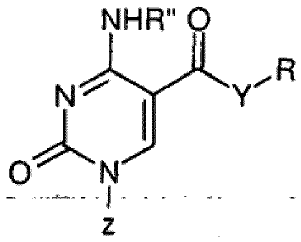 où
Y est choisi dans le groupe constitué par S et NH ; et
R est choisi dans le groupe constitué par les radicaux substitués ou non substitués alkyle en C₁ à C₂₀ (linéaires ou ramifiés), alcényle en C₂ à C₂₀ (linéaires ou ramifiés), aryle, hétérocycliques, et les résidus d'acides aminés naturels et d'acides aminés non naturels, R pouvant éventuellement faire partie d'une structure cyclique qui peut être aromatique, aliphatique ou hétérocyclique ;
R" est choisi dans le groupe constitué par H et acyle ; et
Z est choisi dans le groupe constitué par un ribose, un désoxyribose, un didésoxyribose, et n'importe quelle combinaison de leurs modifications 2', 3' et 5' ;
(B) 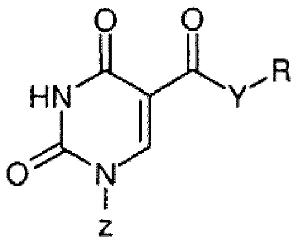 où
Y est choisi dans le groupe constitué par S, NH et NR' ; et
R et R' sont indépendamment choisis dans le groupe constitué par les radicaux substitués ou non substitués alkyle en C₁ à C₂₀ (linéaires ou ramifiés), alcényle en C₂ à C₂₀ (linéaires ou ramifiés), aryle, hétérocycliques, et les résidus d'acides aminés naturels et d'acides aminés non naturels, R et R' pouvant éventuellement faire partie d'une structure cyclique qui peut être aromatique, aliphatique ou hétérocyclique ;
R" est choisi dans le groupe constitué par H et acyle ; et
Z est choisi dans le groupe constitué par un ribose, un désoxyribose, un didésoxyribose, et n'importe quelle combinaison de leurs modifications 2', 3' et 5' ;
et
(C) le groupe constitué par : 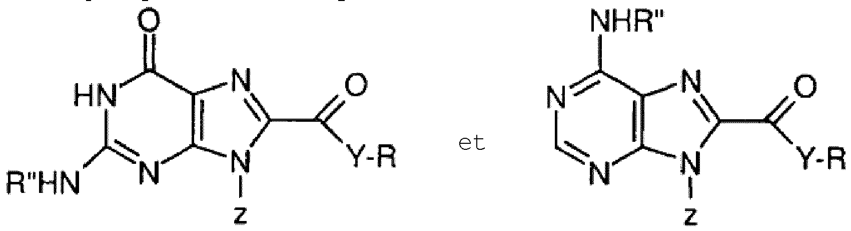 où
Y est choisi dans le groupe constitué par S et NH ; et
R est choisi dans le groupe constitué par les radicaux substitués ou non substitués alkyle en C₁ à C₂₀ (linéaires ou ramifiés), alcényle en C₂ à C₂₀ (linéaires ou ramifiés), aryle, hétérocycliques, et les résidus d'acides aminés naturels et d'acides aminés non naturels, R pouvant éventuellement faire partie d'une structure cyclique qui peut être aromatique, aliphatique ou hétérocyclique ;
R" est choisi dans le groupe constitué par H et acyle ; et
Z est choisi dans le groupe constitué par un ribose, un désoxyribose, un didésoxyribose, et n'importe quelle combinaison de leurs modifications 2', 3' et 5'.

17. Composé selon la revendication 16, dans lequel
R est un alkyle en C₁ à C₂₀ et est (CH₁)ₘ(CH₃)ₙ où 1 vaut 0, 1 ou 2 ; m vaut 0-19 ; n vaut 0, 1, 2 ou 3 ; et dans lequel un ou plusieurs des H sont éventuellement remplacés par =O, -OH, =NH, NH₂, ⁺NMe₃Cl, 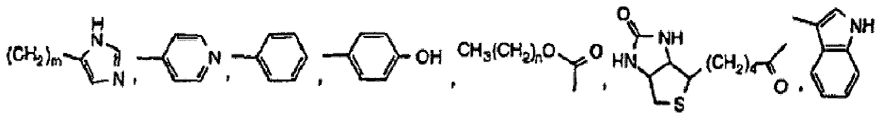 ou un acide aminé,
et, pour les composés (B),
Y est choisi dans le groupe constitué par S et NH.

18. Composé selon la revendication 17, choisi parmi (A), (B) et (C), dans lequel Y est NH et R est un alkyle en C₁ à C₂₀.

19. Composé selon la revendication 18, choisi dans le groupe constitué par

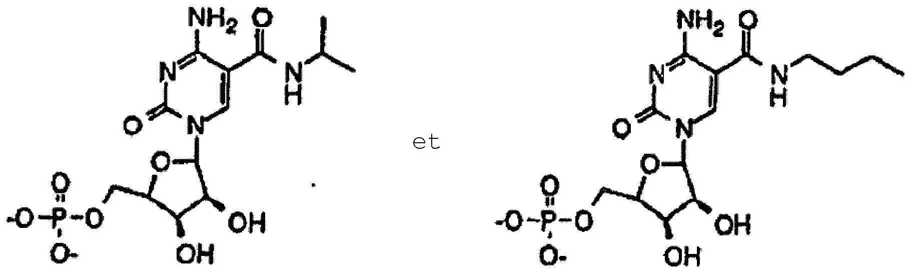

20. Composé selon la revendication 16, choisi dans le groupe constitué par :

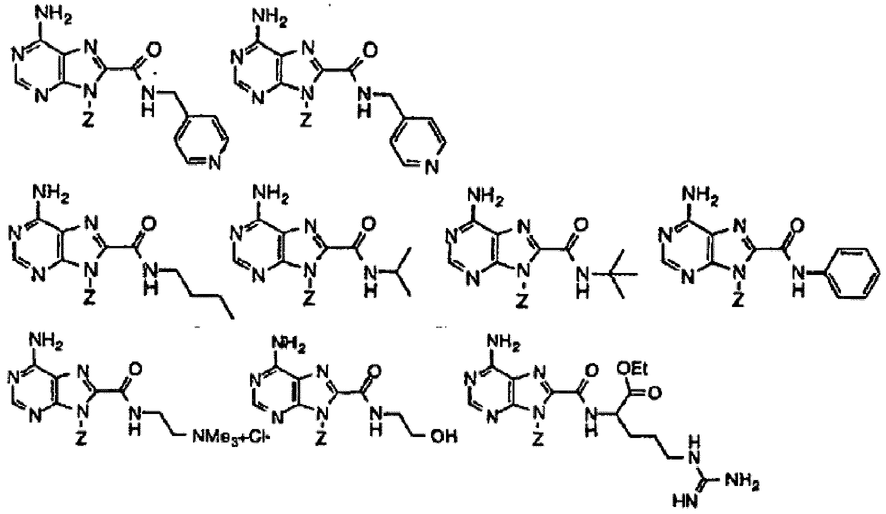

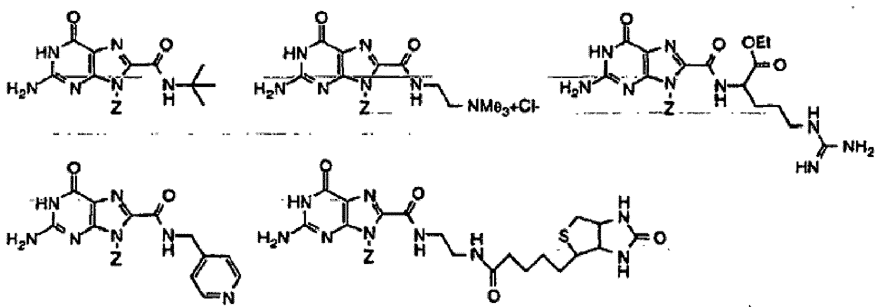

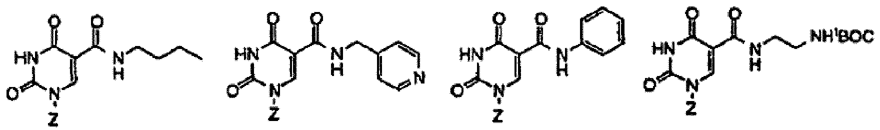

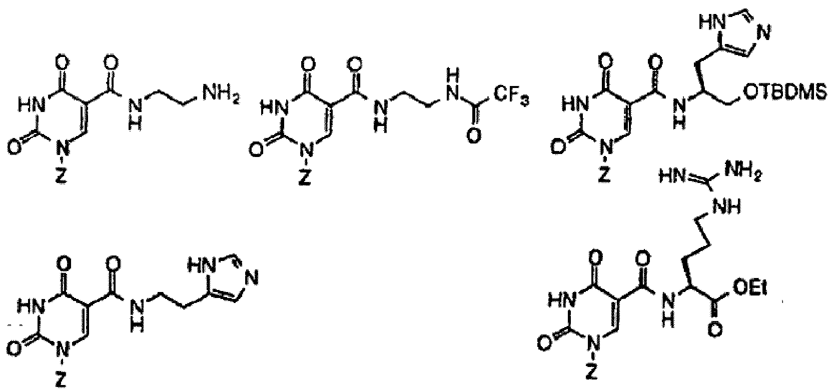

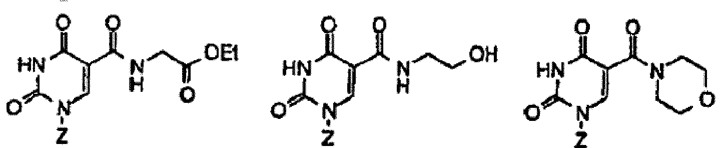

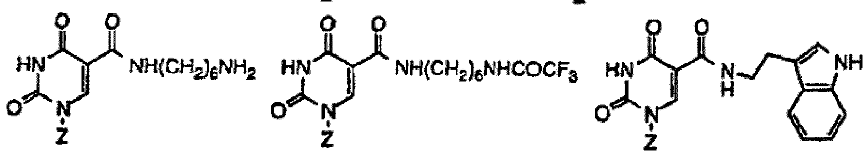

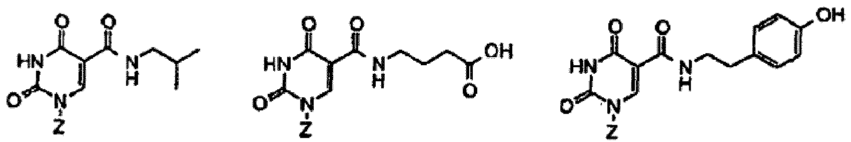

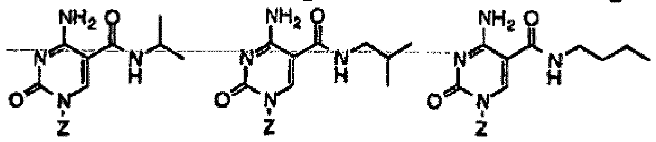

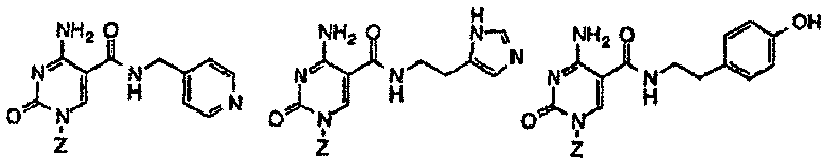

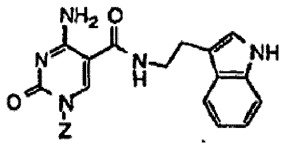

21. Composé selon la revendication 16, choisi dans le groupe constitué par :

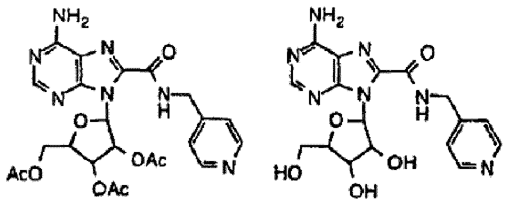

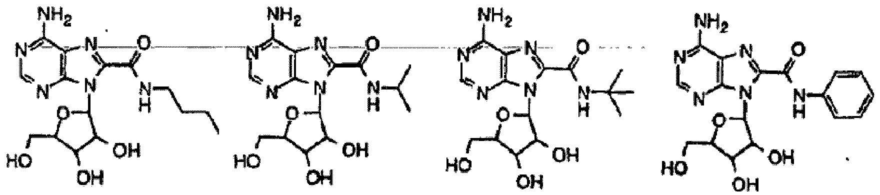

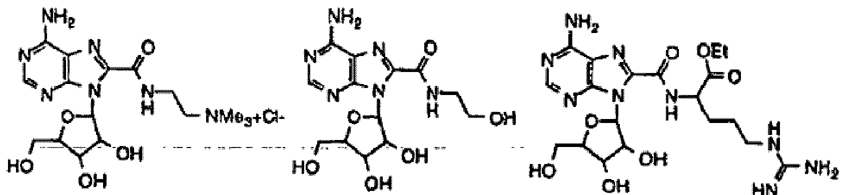

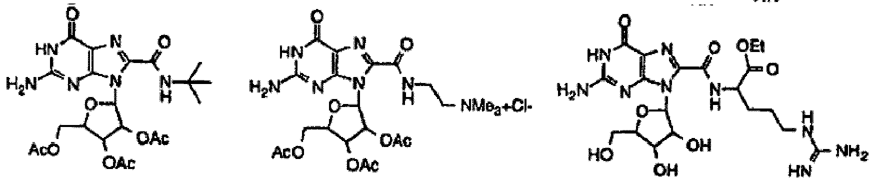

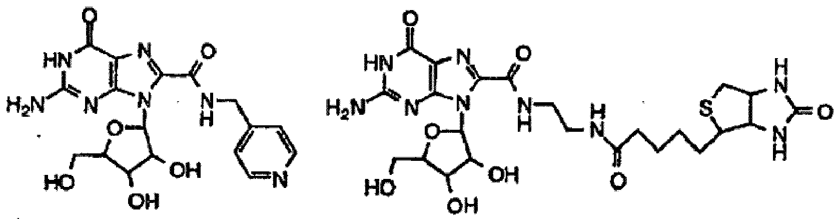

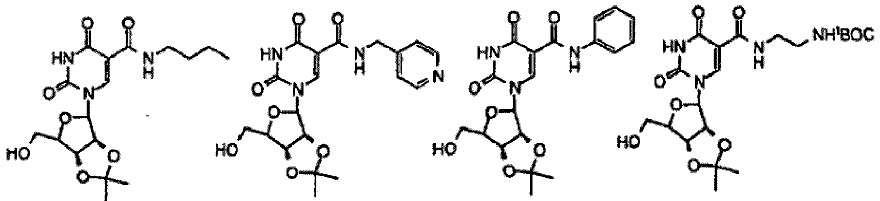

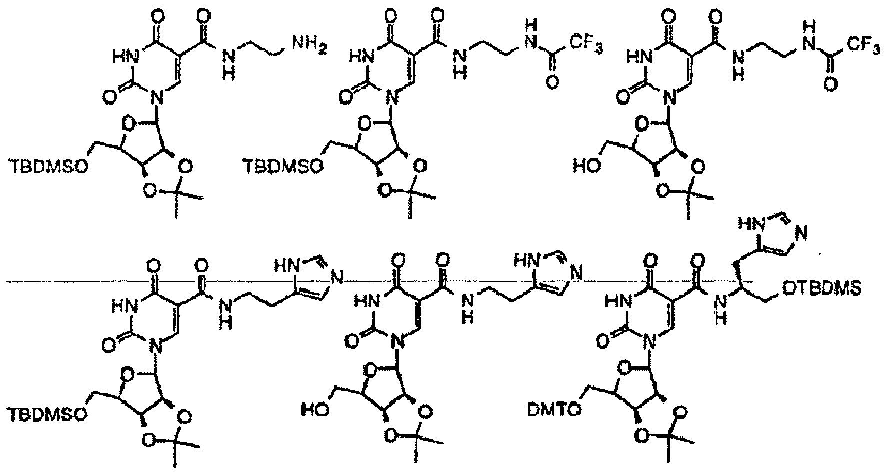

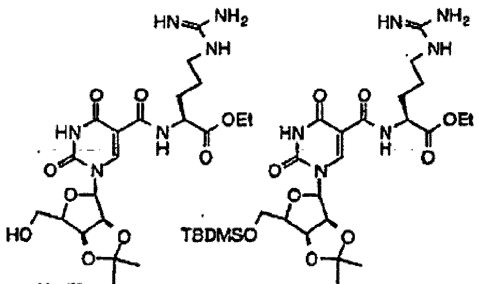

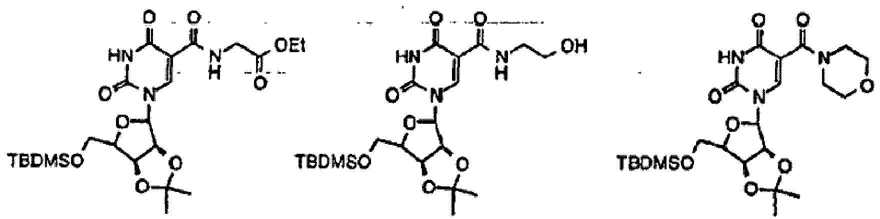

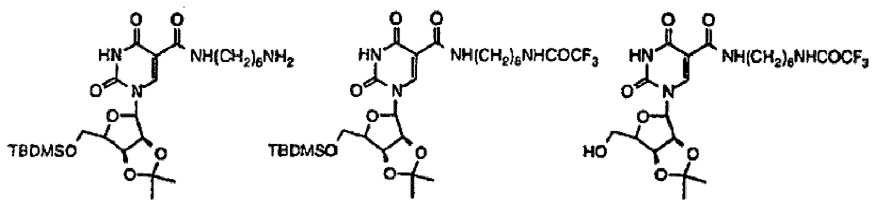

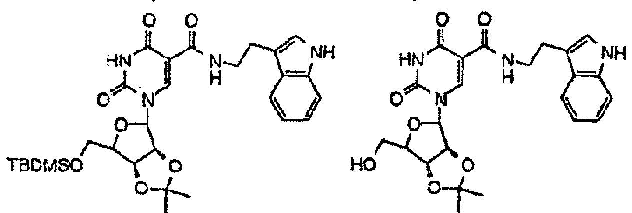

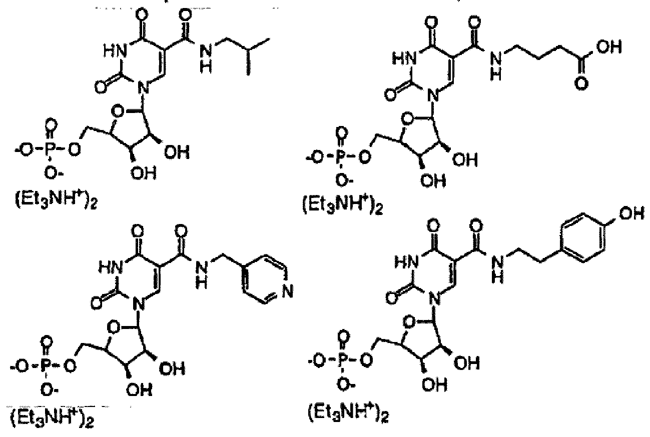

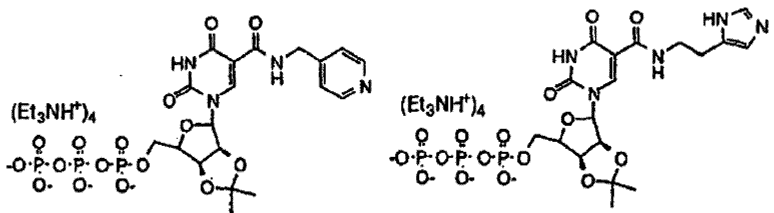

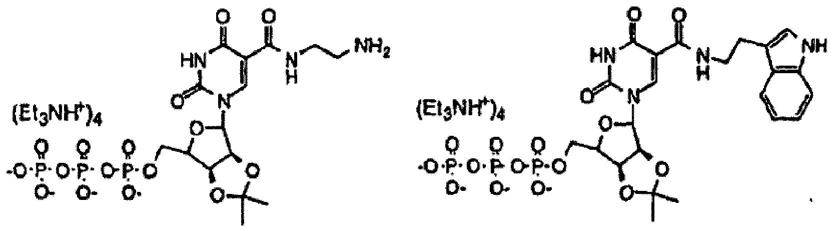

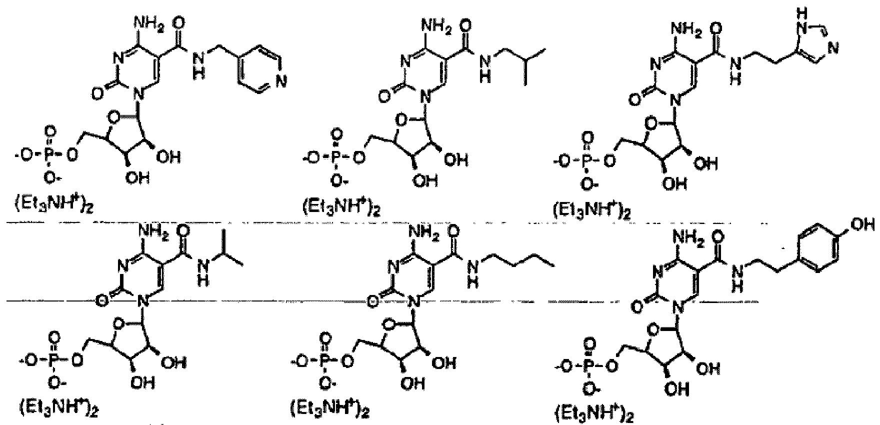

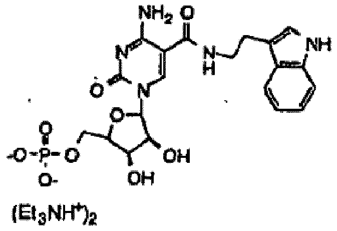

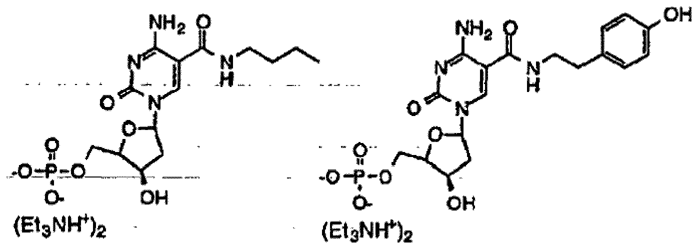

22. Oligoribonucléotide ou oligodésoxyribonucléotide contenant un ou plusieurs nucléosides dérivés d'un nucléoside modifié selon la revendication 16, 17, 18, 19, 20 ou 21.

23. Composé selon la revendication 16, 17, 18, 19, 20 ou 21, pour utilisation en thérapie.

24. Composé selon la revendication 16, 17, 18, 19, 20 ou 21, pour inhiber ou traiter une activité de cytomégalovirus.

25. Utilisation d'un composé selon la revendication 16, 17, 18, 19, 20 ou 21, dans la préparation d'un médicament destiné à être utilisé dans l'inhibition ou le traitement d'une activité de cytomégalovirus.
